# EUROPÄISCHE PATENTSCHRIFT

(11) **EP 4 344 397 B1**
(45) Veröffentlichungstag und Bekanntmachung des Hinweises auf die Patenterteilung: **03.09.2025**
(21) Anmeldenummer: 22748250.2
(22) Anmeldetag: 22.07.2022
(51) Int. Cl.: A01N 1/122

(54) **VERFAHREN ZUR HERSTELLUNG VON ERYTHROZYTENKONZENTRATEN**
METHOD FOR PRODUCING ERYTHROCYTE CONCENTRATES
PROCÉDÉ DE PRODUCTION DE CONCENTRÉS D'ÉRYTHROCYTES

(30) Priorität: 27.07.2021 DE 102021119408
(43) Veröffentlichungstag der Anmeldung: 03.04.2024
(73) Patentinhaber: Blutspendedienst der Landesverbände des Deutschen Roten Kreuzes Niedersachsen, Sachsen-Anhalt, Thüringen, Oldenburg und Bremen g.G.m.b.H., 31832 Springe (DE); Blutspendedienst des Bayerischen Roten Kreuzes, gemeinnützige Gesellschaft mit beschränkter Haftung, 80336 München (DE)
(72) Erfinder: GRAVEMANN, Ute, 30966 Hemmingen (DE); SELTSAM, Axel, 30916 Isernhagen (DE); HANDKE, Wiebke, 31832 Springe (DE)
(74) Vertreter: Müller Schupfner & Partner Patent- und Rechtsanwaltspartnerschaft mbB (Hamburg)
(86) Internationale Anmeldenummer: PCT/DE2022/100535
(87) Internationale Veröffentlichungsnummer: WO 2023/006151

(56) Entgegenhaltungen:
- WO-A1-92/08348
- DE-A1- 102005 062 634
- .: "Gefiltertes Erythrozytenkonzentrat PAGGS-M (UKGM), bestrahlt", GEBRAUCHSINFORMATION UND FACHINFORMATION, 4 April 2018 (2018-04-04), Germany, pages 1 - 7, XP055962710, Retrieved from the Internet <URL:https://portal.dimdi.de/amguifree/am/docoutput/additionalDocDownload.xhtml?dntObjId=6c644824-cbd3-332c-e053-0b0c10ac0cd5> [retrieved on 20220920]
- JOHAN W LAGERBERG ET AL: "Prevention of red cell storage lesion: a comparison of five different additive solutions", BLOOD TRANSFUSION = TRASFUSIONE DEL SANGUE, 1 January 2017 (2017-01-01), Italy, pages 456 - 462, XP055712183, Retrieved from the Internet <URL:https://www.ncbi.nlm.nih.gov/pmc/articles/PMC5589708/pdf/btl-15_456.pdf> DOI: 10.2450/2017.0371-16
- BARDYN MANON: "New routes in additive solution formulations to improve the quality of stored red blood cells", PHD THESIS, 30 August 2019 (2019-08-30), Switzerland, pages 1 - 209, XP055962693, Retrieved from the Internet <URL:https://serval.unil.ch/resource/serval:BIB_62D096E2447A.P001/REF> [retrieved on 20220920]

## Beschreibung

Gegenstand der Erfindung sind ein Verfahren zur Herstellung von mit einer Additivlösung verdünnten Erythrozytenkonzentraten umfassend den Schritt der Bestrahlung mit UV-Licht im Bereich von 300 bis 200 nm sowie die Verwendung der Additivlösung zur Lagerung von Erythrozytenkonzentraten.

### Technisches Umfeld

Erythrozytenkonzentrate (EK) sind aus roten Blutkörperchen (Erythrozyten) bestehende "Blutkonserven". Erythrozytenkonzentrate sind in Deutschland als verkehrsfähige Formen solche, die mindestens 40 g Hämoglobin pro Einheit enthalten und einen Hämatokrit von 0,5 bis 0,7 aufweisen. Der Hämatokrit (Abkürzung: Hkt) bezeichnet den Anteil der roten Blutkörperchen (Erythrozyten) am Volumen des Blutes und versteht sich jeweils im Folgenden in der Einheit L/L. Erythrozytenkonzentrate werden nach dem Stand der Technik üblicherweise mit einer Additivlösung verdünnt und so besser lagerfähig gemacht. Die Additivlösung, häufig auch als Lagerlösung bezeichnet, dient dem Suspendieren und Lagern der Erythrozyten.

Erythrozytenkonzentrate können auf unterschiedliche Weise gewonnen werden. Üblich ist die Gewinnung aus Vollblutspenden oder im Apherese-Verfahren. Im Apherese-Verfahren werden in einem Dialyse-ähnlichen Apparat im Durchflussverfahren Erythrozyten aus dem Spenderblut abgetrennt und die übrigen Blutbestandteile zurück in den Spenderkreislauf geleitet. Für die maschinelle Blutspende wird z.B. das Antikoagulanz ACD-A verwendet.

Vollblutspenden erfolgen, indem mittels venöser Blutentnahme ein bestimmtes Volumen an Vollblut von jeweils einem Spender in jeweils einem Blutbeutel aus einem Kunststoffmaterial verfüllt wird, z.B. 450 mL Vollblut. Der Blutbeutel enthält eine Stabilisatorlösung oder die Stabilisatorlösung wird dem Vollblut hinzugefügt, um die Haltbarkeit des Vollblutes zu erhöhen und dessen Gerinnung entgegenzuwirken.

Eine typische Stabilisatorlösung ist eine CPD-Stabilisatorlösung, umfassend Citrat-Puffer, Natriumdihydrogenphosphat und D-Glukose. Hier werden in der Regel 63 mL CPD-Stabilisatorlösung auf 450 mL Vollblut bzw. 70 mL CPD-Stabilisatorlösung auf 500 mL Vollblut eingesetzt. Durch die Stabilisatorlösung wird der pH-Wert des Vollblutes auf 7,1 bis 7,2 stabilisiert.

Bei der Auftrennung der Vollblutspende in seine Einzelkomponenten werden u.a. die Erythrozytenkonzentrate (EK) gewonnen.

In der Regel wird vor Herstellung der Erythrozytenkonzentrate eine Leukozytendepletion am Vollblut durchgeführt oder die Erythrozytenkonzentrate werden einer Leukozytendepletion unterzogen. Unter Leukozytendepletion versteht man die weitgehende Entfernung der Spender-Leukozyten. In vielen Ländern, wie z.B. in Deutschland, ist dies gesetzlich vorgeschrieben. Die Leukozytendepletion erfolgt z.B., indem das Blut nach Zugabe der Stabilisatorlösung oder das Erythrozytenkonzentrat mit der Additivlösung einen Filter passiert, welches die Leukozyten zurückhält oder z.B. während der Apherese, bei der die Leukozyten durch Zentrifugation von den Erythrozyten getrennt werden. Die verwendeten Filter bestehen häufig aus zu Packungen verpressten Polyesterfasern, so dass Poren definierter Größe entstehen, oder aus einem Polyurethanschwamm mit einer definierten Porengröße.

Die Filtration zur Leukozytendepletion kann unmittelbar nach Gewinnung des Blutes und vor Gewinnung der Erythrozytenkonzentrate oder nach Lagerung der Erythrozytenkonzentrate, ggf. auch erst am Krankenbett vor Verabreichung an einen Patienten erfolgen. Zumindest in Deutschland ist es gängige Praxis, die Leukozytendepletion vor der Lagerung der Erythrozytenkonzentrate durchzuführen.

Zur Herstellung der Erythrozytenkonzentrate wird das Vollblut zentrifugiert. Der Überstand, der das Plasma und den sogenannten Buffy-Coat, bestehend aus Thrombozyten und Leukozyten, enthält, wird abgetrennt und die als Zentrifugat oder Bodensatz verbleibenden Erythrozyten werden in einer Additivlösung aufgeschwemmt.

Ggf. wird das Erythrozytenkonzentrat zuvor noch einmal in einer Nährlösung, die auch unterschiedlich von der Additivlösung sein kann, aufgeschwemmt und erneut zentrifugiert, um diese wieder abzutrennen, sodass Reste des Spender-Blutplasmas durch die Nährlösung ersetzt werden, bevor die Erythrozyten in der Additivlösung aufgeschwemmt und gelagert werden. Diese gewaschenen Erythrozytenkonzentrate sind sinnvoll bei Unverträglichkeiten vorangegangener Transfusionen (z.B. allergische Reaktionen gegen Proteine des Spender-Plasmas, z.B. bei Patienten mit IgA-Mangel).

Um die Erythrozytenkonzentrate, wie sie aus der Zentrifugation erhältlich sind, auf eine physiologisch verträgliche Viskosität einzustellen, muss eine Additivlösung zugesetzt werden, mit der auch für die Erhöhung der Lagerungsfähigkeit notwendige Substanzen zugeführt werden. Die Additivlösung verbessert die Überlebensrate und reduziert die während der Lagerung auftretende Hämolyse der Erythrozyten.

Additivlösungen für Erythrozytenkonzentrate sind an sich bekannt und in verschiedenen Ausgestaltungen bisher vorgeschlagen worden. Aus der US 4267269 ist eine Lösung bekannt, die neben Natriumchlorid, Glucose oder Fructose und Adenin, den Zuckeralkohol Mannit enthält. In der EP 0100419 A2 wird eine Lösung vorgeschlagen, die ebenfalls Natriumchlorid, Glucose oder Fructose und Adenin jedoch als Zuckeralkohol Sorbit bzw. Xylit enthält und ggf. zusätzlich Guanosin. Die EP 0301250 A1 offenbart Additivlösungen, enthaltend Natriumchlorid, Di-Natriumhydrogenphosphat und/oder Natriumdihydrogenphosphat, Glucose und/oder Fructose, Sorbit, Mannit und/oder Xylit, Adenin und/oder Guanosin und ggf. Kolloide. Wirtschaftliche Bedeutung hat z.B. die Additivlösung SAG-M erlangt. Diese enthält Adenin, Glukose, D-Mannitol und Natriumchlorid (C.F. Högman, K. Hedlund, Y. Sahleström: "Red cell preservation in protein-poor media. III. Protection against in vitro hemolysis" in Vox Sang. 1981 Nov-Dec; 41(5-6):274-81).Ein weitere bekannte Additivlösung ist PAGGS-Mannitol (PAGGS-M), die derzeit wie folgt als wässrige Lösung vertrieben wird:

| | |
|---|---|
| 47,4 mmol/L | D-Glukose Monohydrat |
| 8,0 mmol/L | Natriumdihydrogenphosphat Dihydrat |
| 8,0 mmol/L | Dinatriumhydrogenphosphat Dihydrat |
| 1,4 mmol/L | Adenin |
| 1,4 mmol/L | Guanosin |
| 54,9 mmol/L | Mannitol |
| 72 mmol/L | Natriumchlorid |

Diese wird auch in der Gebrauchsinformation und Fachinformation vom April 2018 mit dem Titel "Gefiltertes Erythrozytenkonzentrat PAGGS-M (UKGM), bestrahlt" beschrieben. Weitere Additivlösungen sind aus Johan W. Lagerberg et al.: "Prevention of red cell storage lesions: a comparison of five different additive solutions", Blood transfus 2017, 15, 456-462; Bardyn Manon: "New routes in additive solution formulations to improve the quality of stored red blood cells", Thesis University of Lausanne, 2019, S.1-209 und WO 92/08348 A1 bekannt.

Walter et al. haben in W.H. Walker, M. Netz, K.H. Gänshrit: "49 Tage Lagerung von Erythrozytenkonzentraten in Blutbeuteln mit der Konservierungslösung PAGGS-Mannitol". Beitr. Infusionsther. 26(1990): 55-59 die Lagerfähigkeit von Erythrozytenkonzentraten mit der Additivlösung PAGGS-M untersucht, wobei CPD als Stabilisatorlösung eingesetzt wurde.

Es ist bekannt, dass die therapeutische Anwendung von Blutpräparaten das Risiko birgt, dass die Empfänger des Blutpräparates mit Viren und/oder Bakterien infiziert werden. Genannt seien z.B. die Viren Hepatitis-B (HBV), West-Nil (WNV) und Hepatitis-C (HCV) sowie die Aids-Erreger HIV-1 und HIV-2 oder Bakterien, wie z.B. Staphylokokken oder Streptokokken. Das Risiko besteht immer dann, wenn bei der Herstellung des Präparates kein Schritt zur Inaktivierung bzw. Eliminierung der genannten Pathogene Anwendung findet. Eine Pathogeninaktivierung kann z.B. mit UV-Strahlung durchgeführt werden. Ein derartiges Verfahren ist z.B. aus der WO 2007/076832 A1 bekannt. Ultraviolettes (UV-) Licht wird je nach Wellenlänge differenziert. Im Sinne dieser Anmeldung erfolgt folgende Definition: UVA: 400 bis 320 nm, UVB: 320 bis 280 nm und UVC: 280 bis 200 nm. Es ist bekannt, dass man durch Bestrahlung mit kurzwelligem ultravioletten (UV-) Licht, d.h. im Wellenbereich unterhalb von ca. 320 nm (UVB und UVC), sowohl Viren als auch Bakterien inaktivieren kann, etwa in Blutplasma oder in zellulären Blutpräparaten. Oberhalb von 320 nm ist die Energie der Strahlung zu gering, um Mikroorganismen und Viren effektiv zu inaktivieren. Gegenüber chemischen, photochemischen und photodynamischen Methoden zur Pathogeninaktivierung besitzt die bloße Bestrahlung mit UV-Licht grundsätzlich den Vorteil für sich allein wirksam zu sein und nicht des Zusatzes reaktiver Chemikalien oder photoaktiver Substanzen zu bedürfen.

### Aufgabe der Erfindung

Aufgabe der Erfindung ist es, die Lagerfähigkeit von Erythrozytenkonzentraten zu verbessern, insbesondere für Erythrozyten, die einer UV-Bestrahlung ausgesetzt waren, bevor sie in die Additivlösung eingebracht werden, oder auch ein Medium für die UV-Bestrahlung der Erythrozyten und die Lagerung bereitzustellen.

### Zusammenfassung der Erfindung

Die Erfindung ist durch die unabhängigen Patentansprüche gekennzeichnet, bevorzugte Ausführungsformen sind Gegenstand der Unteransprüche und/oder nachfolgend beschrieben.

Die in dem erfindungsgemäßen Verfahren nach Alternative A eingesetzte Additivlösung umfasst neben Wasser folgende Komponenten:
12 bis 50 mmol/L, insbesondere 17 bis 50 mmol/L oder 20 bis 25 mmol/L, Dinatriumhydrogenphosphat (Na₂HPO₄);
0,1 bis 3,5 mmol/L, insbesondere 1,5 bis 2,5 mmol/L, Adenin;
10 bis 90 mmol/L, insbesondere 45 bis 55 mmol/L, D-Glukose;
0,1 bis 3 mmol/L, insbesondere 1,25 bis 1,75 mmol/L, Guanosin;
10 bis 80 mmol/L, insbesondere 20 bis 60 mmol/L oder sogar 35 bis 45 mmol/L, Natriumchlorid; und
10 bis 50 mmol/L, insbesondere 14 bis 50 mmol/L oder 25 bis 35 mmol/L, Tri-Natriumcitrat.

Insbesondere besteht die Additivlösung aus folgenden Komponenten:
12 bis 50 mmol/L, insbesondere 17 bis 50 mmol/L oder 20 bis 25 mmol/L, Dinatriumhydrogenphosphat (Na₂HPO₄);
0,1 bis 3,5 mmol/L, insbesondere 1,5 bis 2,5 mmol/L, Adenin;
10 bis 90 mmol/L, insbesondere 45 bis 55 mmol/L, D-Glukose;
0,1 bis 3 mmol/L, insbesondere 1,25 bis 1,75 mmol/L, Guanosin;
10 bis 80 mmol/L, insbesondere 20 bis 60 mmol/L oder 35 bis 45 mmol/L, Natriumchlorid;
10 bis 50 mmol/L, insbesondere 14 bis 50 mmol/L oder 25 bis 35 mmol/L, Tri-Natriumcitrat;
wobei der Rest Wasser ist.

Die obigen Komponenten können jeweils auch als ihre Hydrate eingesetzt werden. Die Komponenten liegen in Lösung i.d.R. dissoziiert vor.

Die Additivlösung weist vorzugsweise einen pH von größer 7, vorzugsweise größer 7,5, insbesondere einen pH von 8 bis 9, jeweils bei 22°C, auf.

Die Osmolalität der Additivlösung beträgt vorzugsweise von 260 bis 300 mOsm/kg. Die Messung der Osmolalität erfolgt mittels Gefrierpunkts-Erniedrigungs-Methode (Osmometer).

Gegenstand der Erfindung ist ein Verfahren zur Herstellung von mit der Additivlösung verdünnter Erythrozytenkonzentrate, umfassend den Schritt der Bestrahlung der Erythrozytenkonzentrate und/oder von Vorprodukten der Erythrozytenkonzentrate, wie Vollblut, mit UV-Licht im Bereich von 300 bis 200 nm, insbesondere 280 bis 220 nm, und bevorzugt 260 bis 240 nm (nachfolgend auch allgemein "UV-Bestrahlung" oder "UV-bestrahlt" genannt).

Das Verfahren zur Herstellung von Erythrozytenkonzentraten umfasst in den nachfolgenden drei Ausgestaltungen folgende Schritte:
- Bestrahlung von Vollblut oder verdünntem Vollblut mit UV-Strahlung, Gewinnung eines Erythrozytenkonzentrates aus dem so bestrahlten Vollblut unter Zusatz der Additivlösung oder der Komponenten der Additivlösung;
   oder
- Bestrahlung eines Erythrozytenkonzentrates, umfassend die Additivlösung oder die Komponenten der Additivlösung, unter UV-Bestrahlung, wobei das Erythrozytenkonzentrat vorzugsweise ein verdünntes Erythrozytenkonzentrat mit einem Hkt kleiner 0,5 ist und nach der UV-Bestrahlung auf einen Hkt größer oder gleich 0,5 aufkonzentriert wird;
   oder
- Bestrahlung eines verdünnten Erythrozytenkonzentrates, umfassend eine zweite Additivlösung unter UV-Bestrahlung, wobei das verdünnte Erythrozytenkonzentrat vorzugsweise einen Hkt kleiner 0,5 aufweist und nach der UV-Bestrahlung auf einen Hkt größer 0,5 aufkonzentriert wird,
   wobei die zweite Additivlösung gegen die Additivlösung oder die Komponenten der Additivlösung nach der Bestrahlung zumindest zu größer 75 Gew.%, bezogen auf die zweite Additivlösung, ausgetauscht wird, vorzugsweise vollständig ausgetauscht wird;
wobei die UV-Bestrahlung jeweils mit einer Wellenlänge von 300 bis 200 nm, insbesondere 280 bis 220 nm und bevorzugt 260 bis 240 nm, erfolgt. Werden die Komponenten der Additivlösung zugegeben, meint dies, dass diese so zugegeben werden, dass sich jeweils dieselben Konzentrationen ergeben, als wenn die Additivlösung zugegeben worden wäre. Insofern ist dies nichts anderes als wäre die Additivlösung zugegeben worden.

Weiterhin betrifft die Erfindung somit ein Verfahren mit einem verdünnten Erythrozytenkonzentrat mit einem Hkt von kleiner 0,5 , z.B. mit einem Hkt 0,1 bis 0,4, vorzugsweise 0,25 bis 0,35, enthaltend obige Additivlösung, insbesondere ein leukozytendepletiertes verdünntes Erythrozytenkonzentrat. Das verdünnte Erythrozytenkonzentrat wird z.B. erhalten aus einer 1:2 Verdünnung eines Erythrozytenkonzentrats mit der Additivlösung. Erythrozytenkonzentrate mit einem Hkt von kleiner 0,5 werden vorliegend auch als "verdünnte Erythrozytenkonzentrate" bezeichnet.

Das Erythrozytenkonzentrat ist vorzugsweise wie folgt zusammengesetzt, umfassend (L= Liter):

| | |
|---|---|
| Erythrozyten | 0,40 - 0,80 L/L, insbesondere 0,50 - 0,70 L/L |
| Additivlösung und ggf. | 0,10 - 0,60 L/L, insbesondere 0,25 - 0,50 L/L |
| Stabilisatorlösung | 0,0001 - 0,10 L/L, insbesondere CPD-Stabilisatorlösung |
| Humanplasma | 0,0001 - 0,2 L/L |

Die Summe der Zahlen ergänzt sich jeweils zu einem Zahlenwert von 1 oder kleiner 1 L/L, insbesondere 1 L/L.

Insbesondere ist die Zusammensetzung des Erythrozytenkonzentrats, wenn die UV-Bestrahlung am Vollblut (VB) erfolgte, folgende, umfassend oder bestehend aus:

| | |
|---|---|
| Erythrozyten | 0,5 - 0,7 L/L |
| Additivlösung | 0,27 - 0,48 L/L |
| CPD-Stabilisatorlösung | 0,03 - 0,09 L/L |
| Humanplasma | 0,025 - 0,15 L/L oder 0,015 - 0,025 L/L |

oder nach UV- Bestrahlung des Erythrozytenkonzentrats, wenn die UV-Bestrahlung nicht am Vollblut (VB) erfolgte, umfassend oder bestehend aus:

| | |
|---|---|
| Erythrozyten | 0,5 - 0,7 L/L |
| Additivlösung | 0,27 - 0,49 L/L |
| CPD-Stabilisatorlösung | 0,001 - 0,009 L/L oder 0,001 - 0,014 L/L |
| Humanplasma | 0,005 - 0,05 L/L |

Eine geeignete CPD-Stabilisatorlösung ist nach einer Ausführungsform wie folgt aufgebaut, enthaltend:

| | |
|---|---|
| Tri-Natriumcitrat Dihydrat | 80 bis 100 mmol/L insbesondere 89,4 mmol/L |
| Citronensäure Monohydrat | 13 bis 18 mmol/L, insbesondere 15,6 mmol/L |
| NaH₂PO₄ Dihydrat | 13 bis 19 mmol/L, insbesondere 16,1 mmol/L |
| D-Glucose Monohydrat | 115 bis 140 mmol/L, insbesondere 128,7 mmol/L |

Die CPD-Stabilisatorlösung wird nach einer Ausführungsform dem Vollblut im Volumenverhältnis 1 : 6,1 bis 1 : 8,1 insbesondere 1: 7,14 (jeweils V/V), z.B. 63 mL CPD-Stabilisatorlösung auf 450 mL Vollblut oder 70 mL CPD-Stabilisatorlösung auf 500 mL, zugesetzt und zwar bevor die Additivlösung eingesetzt wird. Entsprechend der Aufarbeitung verbleiben dann die obigen Bestandteile der CPD-Stabilisatorlösung in bestimmten Konzentrationen im Erythrozytenkonzentrat.

Da Glukose und Tri-Natriumcitrat auch in der Stabilisatorlösung enthalten sind, erhöht sich deren Anteil im Erythrozytenkonzentrat entsprechend, wenn CPD-Stabilisatorlösung eingesetzt wird. Es ist aber auch möglich, andere Stabilisatorlösungen einzusetzen.

Nach einer Ausführungsform enthält das Erythrozytenkonzentrat:
10 bis 30 mmol/l, insbesondere 14 bis 26 mmol/l, D-Glukose;
5 bis 12 mmol/l, insbesondere 6 bis 10 mmol/l, Dinatriumhydrogenphosphat, z.B. als Dihydrat;
0,3 bis 1,2 mmol/L, insbesondere 0,5 bis 0,8 mmol/l, Adenin
0,25 bis 0,9 mmol/L, insbesondere 0,4 bis 0,7 mmol/L, Guanosin;
8 bis 25 mmol/L, insbesondere 11 bis 20 mmol/L, Natriumchlorid;
6 bis 18 mmol/L, insbesondere 8 bis 16 mmol/L, Tri-Natriumcitrat;
und fakultativ
0,01 bis 1 mmol/L, insbesondere 0,02 bis 0,8 mmol/L, Natriumdihydrogenphosphat, z.B. als Dihydrat;
0,01 bis 1 mmol/L, insbesondere 0,02 bis 0,8 mmol/L, Citronensäure, z.B. als Monohydrat.

Diese Ausführungsform ist z.B. erhältlich, wenn das Erythrozytenkonzentrat durch Zugabe von CPD- oder CPDA-1 -Stabilisatorlösung zur Blutspende und erfindungsgemäßer Additivlösung erhalten wurde, wobei dann das Natriumdihydrogenphosphat und die Citronensäure durch die CPD-Stabilisatorlösung eingetragen werden.

Weiterhin betrifft die Erfindung somit die Verwendung der Additivlösung zur Lagerung von Erythrozytenkonzentraten.

Der Unterschied in der Konzentration der CPD-Stabilisatorlösung und des Humanplasmas ergibt sich durch Verdünnung des EK mit der Additivlösung und anschließende Aufkonzentration. Beim Aufkonzentrieren wird ein Teil des Überstandes entfernt und damit auch ein Teil des ursprünglich enthaltenen Plasmas und der CPD-Stabilisatorlösung.

### Detaillierte Beschreibung der Erfindung

Erythrozytenkonzentrate werden nach einer Ausführungsform aus einzelnen Blutspenden isoliert oder aber durch maschinelle Apherese von einzelnen Spendern gewonnen. Das Volumen der Präparate liegt im Allgemeinen zwischen ca. 200 und 350 mL. Das Volumen von Vollblutspenden liegt meist zwischen 400 und 500 mL. Die Präparate werden jeweils in flachen Kunststoffbeuteln im Allgemeinen bei ca. 4°C bis 37°C für Vollblut und 4°C +/- 2°C für Erythrozytenkonzentrat gelagert.

Die Leukozytendepletion kann auf der Stufe des Vollblutes durchgeführt werden, d.h. vor dem ersten Zentrifugieren des Vollblutes, oder auf der Stufe, wo die durch Zentrifugieren gewonnenen Erythrozyten in der Additivlösung aufgeschwemmt und verdünnt sind, d.h. auf der Stufe der Erythrozytenkonzentrate. Die Leukozytendepletion erfolgt in der Regel durch Filtration. Bei der Gewinnung der Erythrozyten mittels Apherese ist eine gesonderte Leukozytendepletion nicht notwendig, die Leukozytendepletion erfolgt hier bereits in Rahmen der Apherese.

Nach einer Ausgestaltung erfolgt die Durchführung der Leukozytendepletion an einem mit der erfindungsgemäßen Additivlösung verdünnten Erythrozytenkonzentrat bei einem Hkt von z.B. 0,1 bis 0,4. Nach der Leukozytendepletion erfolgen dann ggf. eine UV-Bestrahlung und danach eine Aufkonzentration auf einen Hkt von insbesondere 0,5 bis 0,7. Die Leukozytendepletion kann vor oder nach UV-Bestrahlung erfolgen, bevorzugt vorher.

Die UV- Bestrahlung kann am Vollblut (VB), d.h. vor der Herstellung des Erythrozytenkonzentrates, oder am Erythrozytenkonzentrat erfolgen. Als Produkt wird ein UV-behandeltes, pathogen-abgereichertes Erythrozytenkonzentrat und ggf. leukozytendepletiertes Erythrozytenkonzentrat in der erfindungsgemäßen Additivlösung erhalten.

Ausgangsmaterial für die UV-Bestrahlung von Erythrozytenkonzentraten sind z.B. Erythrozytenkonzentrate mit einem Hkt zwischen 0,8 bis 1, insbesondere zwischen 0,8 und 0,98. Diese werden mittels der erfindungsgemäßen Additivlösung nach einer Ausführungsform auf einen Hkt von 0,5 bis 0,7 eingestellt und bei dieser Konzentration mit UV oder bevorzugt bei weiterer Verdünnung (verdünntes Erythrozytenkonzentrat) bestrahlt. Im Sinne der vorliegenden Anmeldung wird ein Erythrozytenkonzentrat mit einem Hkt kleiner 0,5 als verdünntes Erythrozytenkonzentrat bezeichnet.

Das leukozytendepletierte Erythrozytenkonzentrat kann auch auf einen Hkt von 0,1 bis 0,4, vorzugsweise 0,25 bis 0,35, (verdünntes Erythrozytenkonzentrat) durch weitere Verdünnung mit der Additivlösung, z.B. aus einer 1:2 Verdünnung des Erythrozytenkonzentrates mit Additivlösung, eingestellt werden. Die so verdünnten Erythrozytenkonzentrate werden über eine sterile Schlauchverbindung in einen Bestrahlungsbeutel überführt und unter heftiger Bewegung mit UV-Licht bestrahlt.

Das bestrahlte Erythrozytenkonzentrat wird in einen Leerbeutel überführt und auf einen Hkt von 0,5-0,8, insbesondere 0,5-0,7, aufkonzentriert, z.B. durch Zentrifugation und Abpressen des Überstands.

Es ist bekannt, dass man durch Bestrahlung mit kurzwelligem ultravioletten (UV-) Licht Pathogene in Blutprodukten inaktivieren kann. Dies erfolgt nach dem Verfahren der Erfindung, indem die Blutprodukte einer Bestrahlung mit ultraviolettem (UV-) Licht bei Wellenlängen von 300 bis 200 (Bereich UVB bis UVC) ausgesetzt werden, insbesondere einer Bestrahlung bei Wellenlängen im UVC Bereich von 280 nm bis 220 nm, insbesondere 260 bis 240 nm.

Die Strahlungsenergie beträgt vorzugsweise von 0,3 bis 10 J/cm², weiter bevorzugt 2,5 bis 5,5 J/cm² und besonders bevorzugt 3 bis 5 J/cm² für Vollblut und bei Erythrozytenkonzentraten von vorzugsweise 1,5 bis 4,5 J/cm² und besonders bevorzugt 2 bis 4 J/cm² (jeweils bezogen auf die Strahlungsenergie, die auf das Blutprodukt einwirkt). Die Strahlungsenergie, die auf das Behältnis wie einen Bestrahlungsbeutel einwirkt, ist tatsächlich größer, weil der Bestrahlungsbeutel je nach Material typischerweise Strahlungsenergie der relevanten Wellenlänge absorbiert.

Wenn die Bestrahlung durch ein Medium erfolgt, das UV-Strahlung absorbiert, ist die Strahlungsenergie entsprechend zu erhöhen. Bestrahlungsbeutel aus EVA (Ethylenvinylacetat) absorbieren z.B. etwa 30 bis 40% der Strahlungsenergie. Die Bestrahlung erfolgt insbesondere bei einer Temperatur des Blutproduktes von 2 bis 37°C.

Ein derartiges Verfahren zur UV-Bestrahlung ist z.B. aus der WO 2007/076832 A1 bekannt und wird auf die Erythrozytenkonzentrate nach der vorliegenden Erfindung angewandt. Hiernach werden die Präparate, d.h. Spenderblut (Vollblut) und/oder Erythrozytenkonzentrate (EKs) in einem Bestrahlungsbeutel in geeigneter Weise bewegt, so dass eine ständige Umwälzung der Proben im Behältnis erfolgt. Die Bewegung erfolgt hierbei so heftig, dass sich innerhalb der Flüssigkeit bzw. Suspension bereichsweise Schichten ausbilden, die so dünn sind, dass sie vom eingesetzten Licht durchdrungen werden können. Die Bewegung erfolgt so, dass Flüssigkeit bzw. Suspension im Beutel wirksam vermischt wird. Beides wird insbesondere realisiert, wenn u.a. folgende Voraussetzungen gegeben sind:
1. Die Bestrahlungsbeutel sind flexibel.
2. Die Bestrahlungsbeutel sind zu maximal 40 %, insbesondere maximal 30 %, insbesondere zu maximal 15%, des maximalen Füllvolumens gefüllt.
3. Die Beutel werden heftig bewegt, z.B. entweder horizontal (linear in Hin- und Her-Richtung bzw. kreis- oder ellipsenförmig) und/oder aber vertikal (gewippt).

In Verbindung mit der ständigen Durchmischung, die gleichzeitig stattfindet, wird letztendlich das gesamte Präparat (und die darin enthaltenen Pathogene) bestrahlt, und es ist somit pathogenreduziert.

Die Bestrahlungsbeutel können mit einem Orbitalschüttler, Plattformschüttler, Wippschüttler oder Taumelschüttler geschüttelt werden und werden vorzugsweise während zumindest dreiviertel der gesamten Bestrahlungsdauer bewegt. Die Bestrahlungsbeutel haben typischerweise ein Volumen von bis zu 5000 mL. Wenn die Bestrahlungsbeutel einseitig aufgelegt sind, ändert sich während und durch das Bewegen bzw. Schütteln die Höhe des Bestrahlungsbeutels ständig über die gesamte obere Fläche des Bestrahlungsbeutels, die mit dem Beutelinhalt in Kontakt steht, bezogen auf die Distanz entlang der Flächennormale zwischen Fläche, auf der der Bestrahlungsbeutel aufliegt, und Schnittpunkt mit der oberen Fläche des Bestrahlungsbeutels.

Die Bestrahlungsbeutel sind aus UV-transparentem Kunststoffmaterial gefertigt. Geeignete Kunststoffe sind z.B. Ethylenvinylacetat und Polyolefine mit z.B. Folienstärken von 1 mm und weniger, insbesondere Folienstärken kleiner 0,5 mm. Die Bestrahlungsbeutel sind flächig ausgebildet und weisen vorzugsweise keine Absorptionsmaxima im Bereich von 200 bis 320 nm auf. Die Bestrahlungsbeutel sind im liegenden gefüllten Zustand nur einige mm dick, z.B. kleiner 10 mm und insbesondere 5 mm, vorzugsweise sogar kleiner 3 mm und sind bestimmt, Probenvolumina von z.B. bis zu 600 mL aufzunehmen. Das maximale Fassungsvermögen (Volumen) des Bestrahlungsbeutels ist vorzugsweise aber um mindestens Faktor 3, i.d.R. um mindestens Faktor 5, größer oder mindestens 10 als das tatsächliche in ihm enthaltene zu behandelnde Probenvolumen. Beispielhaft hat der Bestrahlungsbeutel liegend eine Grundfläche von 19 x 38 cm und ein Füllvolumen von 500 bis 600 mL, womit sich im liegenden und ruhenden Zustand eine mittlere Füllhöhe von 6,9 bis 8,3 mm ergibt.

Jede UV-bestrahlte Einheit ist vorzugsweise auf einen Spender zurückzuführen.

Hämatokrit (Hkt) bezeichnet den Anteil der zellulären Bestandteile im Blut. Normale Hkt-Werte im Blut liegen bei Männern zwischen 0,42 und 0,5 und bei Frauen zwischen 0,37 und 0,45. Vorliegend ist dieser in L/L angegeben. Da die Erythrozyten physiologisch 99 % des Gesamtvolumens der Blutzellen darstellen, entspricht der Hkt-Wert ungefähr dem Anteil des Zellvolumens.

Bestimmt wird der Hkt durch Zentrifugieren einer gerinnungsfreien Blutprobe in einem Röhrchen nach DIN 58933-1:1995-01. Die Gerinnung des Blutes wird dabei durch Zugabe von Antikoagulantien wie EDTA (Ethylendiamintetraacetat) oder Heparin verhindert. Die schwereren roten Blutkörperchen setzen sich vom Plasma ab, die Höhe der Erythrozytensäule wird im Verhältnis zur gesamten Blutsäule gemessen. Die Grenzen zwischen Erythrozyten, Leukozyten/Thrombozyten und Blutplasma sind mit bloßem Auge erkennbar. Sind die Leukozyten und/oder Thrombozyten und Blutplasma bereits abgetrennt, besteht der sich absetzende Festkörper fast ausschließlich aus Erythrozyten.

### Experimenteller Teil

Einfluss der Additivlösung UG65 auf die Qualität der UVC-bestrahlten Blutpräparate sowie Effektivität der Pathogeninaktivierung von Blutpräparaten mittels UV-Bestrahlung.

### Herstellung der Komponenten

Vollblutspenden (450 - 500 mL) wurden in 70 mL des Antikoagulanz CPD gesammelt (Tag 0), nachfolgend gemeinsam Vollblutspende genannt, und über Nacht bei Raumtemperatur gelagert. Das Antikoagulanz CPD enthielt neben Wasser folgende Bestandteile:

| | mmol/L |
|---|---|
| Tri-Natriumcitrat Dihydrat | 89,4 |
| Citronensäure Monohydrat | 15,6 |
| NaH₂PO₄ Dihydrat | 16,1 |
| D-Glucose Monohydrat | 128,7 |

Am Tag 1 wurde das Vollblut direkt für Pathogeninaktivierungsexperimente verwendet oder zum Erythrozytenkonzentrat weiterverarbeitet. Dazu wurden die Erythrozyten nach Zentrifugation in einer herkömmlichen Beutelzentrifuge und automatischer Komponententrennung durch einen Abpressautomaten als "trockenes" Erythrozytenkonzentrat gewonnen und anschließend in der jeweils angegebenen Additivlösung (110 mL) aufgeschwemmt. Die Abreicherung der Leukozyten erfolgte mittels Filtration auf der Stufe des Vollbluts ("Vollblutfiltration") oder des Erythrozytenkonzentrates (Filtration nach Zentrifugation und Abpressen) mittels eines Leukozytendepletionsfilter.

### Zusammensetzung der Additivlösung UG65:

Die wässrige Lösung enthält die folgenden Bestandteile:

| | |
|---|---|
| 22,7 mmol/L | Dinatriumhydrogenphosphat Dihydrat |
| 1,85 mmol/L | Adenin |
| 51,6 mmol/L | D-Glukose Monohydrat |
| 1,44 mmol/L | Guanosin |
| 40 mmol/L | Natriumchlorid |
| 28,4 mmol/L | Tri-Natriumcitrat Dihydrat |
| Rest | Wasser |

### Zusammensetzung der Additivlösung SAG-M (Verwendung nicht erfindungsgemäss):

Die wässrige Lösung enthält die folgenden Bestandteile:

| | |
|---|---|
| 1,25 mmol/L | Adenin |
| 45,4 mmol/L | D-Glukose Monohydrat |
| 28,8 mmol/L | D-Mannitol |
| 150 mmol/L | Natriumchlorid |
| Rest | Wasser |

### Pathogeninaktivierung mittels UV-Bestrahlung

Vollblut (520 - 570 mL) oder in Additivlösung verdünntes Erythrozytenkonzentrat (600 mL, Hkt ca. 0,3) wurden in einen UV-durchlässigen Beutel (19 x 38 cm Grundfläche aus EVA) gefüllt und auf einer UV-Bestrahlungsanlage (Macotronic UV) mit UVC-Licht (254 nm) mit einer UVC-Dosis von 4,5 J/cm² (EK) bzw. 6 J/cm² (VB) bestrahlt und gleichzeitig geschüttelt (300 rpm).

Aus dem Vollblut oder dem verdünnten Erythrozytenkonzentrat wurde anschließend mittels Zentrifugation und automatischer Separation ein herkömmliches Erythrozytenkonzentrat mit einem Hkt von 0,5 bis 0,7 gewonnen.

Die Angaben zur eingestrahlten Energie wirken auf das Äußere des Bestrahlungsbeutels ein. Den Bestrahlungsbeutel durchdringt ca. 50 bis 75 %, vorliegend geschätzt 60% der eingestrahlten Energie. Der Bestrahlungsbeutel wurde von oben und von unten bestrahlt.

### Bestimmung der Qualitätsparameter

Die Hämolyserate [%] ist definiert als der prozentuale Anteil des freien Hämoglobins im Überstand der Erythrozytenkonzentrate im Vergleich zum Gesamtgehalt. Hämolyse (%) = ((100-Hämatokrit*100) x freies Hämoglobin im Überstand / Ge- samthämoglobin).

Der Hkt wurde mittels Hämatokritzentrifuge (Haematokrit 210, Hettich) bestimmt. Freies Hämoglobin im Überstand wurde photometrisch mit der 3-Wellenlängenmethode nach Harboe bestimmt (vergleiche: M. Harboe, A method for determination of hemoglobin in plasma by near-ultraviolet spectrophotometry. Scand J Clin Lab Invest, 1959. 11(1): p. 66-70). Gesamthämoglobin wurde mittels Hämatologieautomat (XS1000i oder XN550, Sysmex) gemessen.

Die Bestimmung der Glukose- und Laktatkonzentration erfolgte mit dem Blutgasanalysator ABL90 FLEX (Radiometer). Der ATP-Gehalt der Erythrozyten wurde mit dem kommerziell erhältlichen Kit ATP Hexokinase FS (DiaSys Greiner) gemessen. Der pH wurde bei 22°C an einem herkömmlichen pH-Meter bestimmt. Das Volumen wurde durch Wägen unter Berücksichtigung der spezifischen Dichte des Erythrozytenkonzentrats ermittelt.

### Versuch 1

### Qualitätsparameter in UG65 UVC-bestrahlter und in UG65 gelagerter Erythrozytenkonzentrate

Erythrozytenkonzentrate (n=9) in Additivlösung UG65 wurden wie oben beschrieben UVC-bestrahlt und wieder aufkonzentriert. Die fertigen Erythrozytenkonzentrate mit einem Hkt von ca. 0,6 wurden anschließend bei 4 ± 2°C gelagert und wöchentlich wurden Proben zur Bestimmung der in vitro Qualität entnommen.

### Ergebnisse

Die UVC-bestrahlten Erythrozytenkonzentrate wiesen einen Hkt zwischen 0,59 und 0,64 auf und entsprachen damit den Guidelines des Council of Europe. Die Hämolyserate stieg im Verlauf der Lagerung an. Am Tag 36 der Lagerung zeigten aber alle neun Erythrozytenkonzentrate eine Hämolyserate von unter 0,8 % und entsprachen damit den Qualitätsanforderungen der Guideline des Council of Europe.

Der pH-Wert der UVC-bestrahlten Erythrozytenkonzentrate lag bei 7,14 ± 0,06 an Tag 2 und sank im Verlauf der Lagerung auf 6,54 ± 0,05 an Tag 36. Parallel dazu fiel der Glukosegehalt der Erythrozytenkonzentrate von 37,8 ± 1,6 auf 23,7 ± 1,9 und die Laktatkonzentration nahm von 6,9 ± 0,6 auf 30,4 ± 1,6 zu.

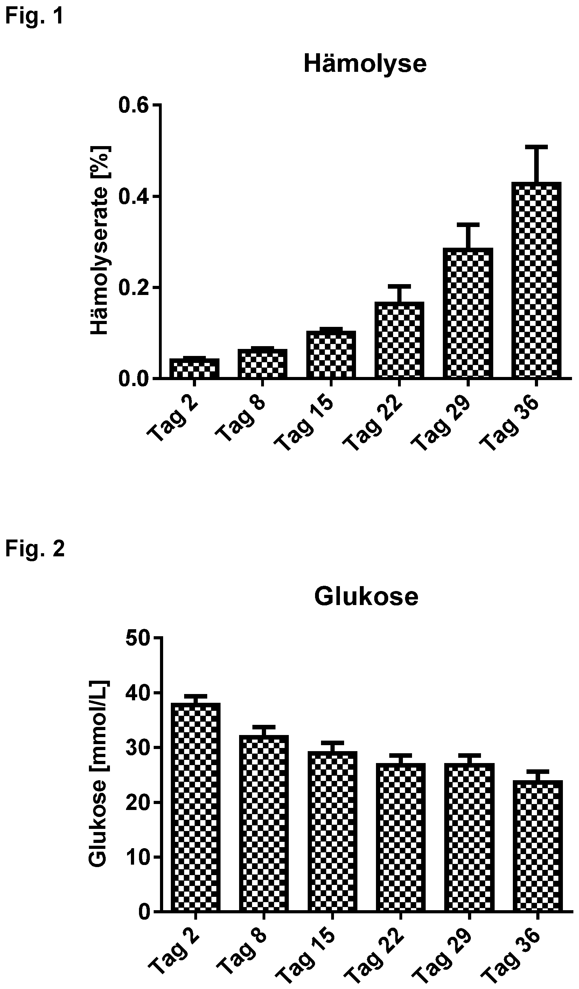

**Fig. 1** zeigt die Hämolyserate im Verlauf der Lagerung als Funktion der Zeit in Tagen.

**Fig. 2** zeigt die Abnahme der Glukosekonzentration der in UG65 UVC-bestrahlten und in UG65 gelagerten Erythrozytenkonzentrate und **Fig. 3** die Zunahme der Laktatkonzentration der in UG65 UVC-bestrahlten und in UG65 gelagerten Erythrozytenkonzentrate im Verlauf der Lagerung.

Die Werte sind jeweils als Mittelwert aus 9 Proben angegeben.

Es konnte festgestellt werden, dass die neu entwickelte Additivlösung geeignet zur Herstellung von UVC-bestrahlten Erythrozytenkonzentraten in einer guten Qualität ist.

### Versuch 2:

### Einfluss unterschiedlicher Additivlösungen während der UVC-Bestrahlung bei nachfolgender Lagerung in der Additivlösung UG65

Vier trockene Erythrozytenkonzentrate wurden gepoolt und wieder aufgeteilt. Ein Erythrozytenkonzentrat wurde mit 110 mL UG65 aufgeschwemmt und zur Leukozytendepletion filtriert (unbehandelte Kontrolle ohne UVC-Bestrahlung).

Für die drei übrigen Erythrozytenkonzentrate wurde jeweils ein gepooltes Erythrozytenkonzentrat mit 110 mL isotoner Kochsalzlösung (NaCl 0,9%), mit 110 mL Additivlösung SAG-M und mit 110 mL Additivlösung UG65 aufgeschwemmt, filtriert und anschließend auf einen Hkt von ca. 0,3 mit der jeweils gleichen Additivlösung verdünnt. Die UVC-Bestrahlung erfolgte wie oben angegeben mit einer UVC-Dosis von 4,5 J/cm². Anschließend wurden die UVC-bestrahlten Erythrozytenkonzentrate zentrifugiert, der Überstand wurde abgenommen und die Erythrozyten wurden alle jeweils in Additivlösung UG65 wieder aufgeschwemmt. Die Lagerung der Erythrozytenkonzentrate in der Additivlösung UG65 erfolgte bei 4 ± 2°C und wöchentlich wurden Proben zur Bestimmung der in vitro Qualität entnommen.

### Ergebnisse

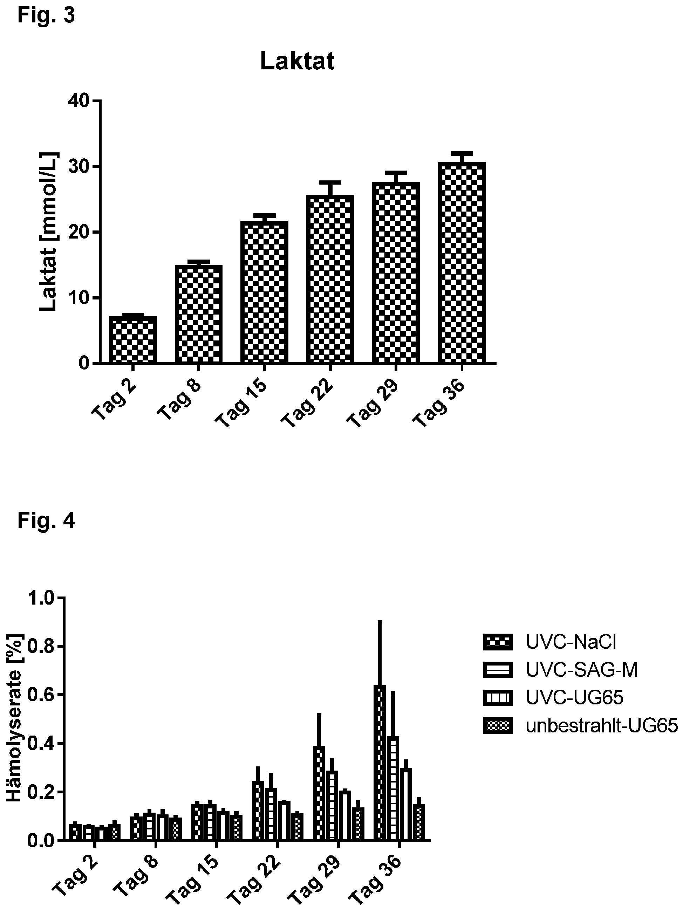

Wie in Fig. 4 gezeigt, führte die UVC-Bestrahlung im Vergleich zur unbestrahlten Kontrolle zu einer erhöhten Hämolyserate. Die Hämolyserate war am höchsten, wenn die Erythrozyten in Anwesenheit von NaCl oder SAG-M bestrahlt wurden. Die beste Qualität der UVC-bestrahlten Erythrozytenkonzentrate wurde erreicht, wenn die Additivlösung UG65 bereits bei der Bestrahlung anwesend war.

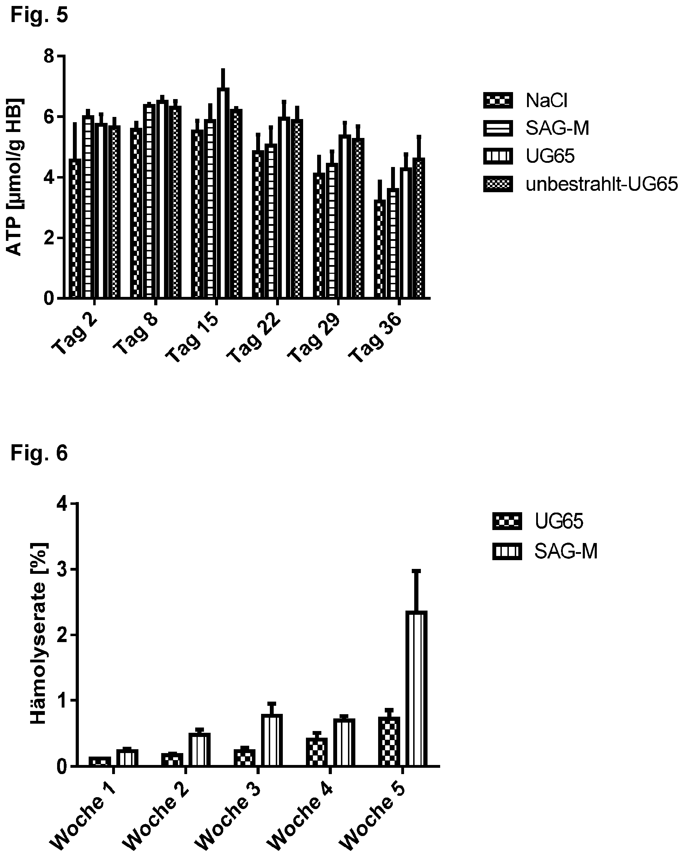

Der ATP-Gehalt als Parameter für den Energiestatus der Erythrozyten wurde ebenfalls durch die UVC-Bestrahlung beeinflusst. Der ATP-Gehalt am Ende der Lagerung war am höchsten, wenn die Erythrozyten in Anwesenheit von UG65 bestrahlt wurden (Fig. 5). Die Bestrahlung der Erythrozytenkonzentrate in Anwesenheit von NaCl oder SAG-M führte zu einer Abnahme des ATP-Gehalts im Vergleich zur unbestrahlten Kontrolle.

**Fig. 4** gibt die Hämolyserate und **Fig. 5** den ATP-Gehalt der UVC-bestrahlten Erythrozytenkonzentrate an, die in Anwesenheit von NaCl, SAG-M oder UG65 bestrahlt wurden und sodann in UG65 gelagert wurden. Als Kontrolle diente ein EK, das ohne UVC- Bestrahlung in UG65 gelagert wurde.

Aus der Versuchsserie geht hervor, dass die Verdünnung der Erythrozytenkonzentrate mit der Additivlösung UG65 während der UVC-Bestrahlung einen positiven Effekt auf die Qualität der Erythrozyten hat. Die neu entwickelte Additivlösung bietet einen Vorteil gegenüber anderen möglichen Verdünnungslösungen wie Kochsalzlösung oder herkömmlichen Additivlösungen.

### Versuch 3:

### Vergleich der Qualität von UVC-behandelten Erythrozytenkonzentraten bestrahlt und gelagert in der Additivlösung UG65 im Vergleich zu Erythrozytenkonzentraten bestrahlt und gelagert in der konventionellen Additivlösung SAG-M

"Trockenes" Erythrozytenkonzentrat (Hämatokrit > 0,8) wurde wie oben beschrieben gewonnen. Zwei trockene Erythrozytenkonzentrate wurden gepoolt und wieder aufgesplittet und anschließend einmal in 110 mL der handelsüblichen Additivlösung SAG-M (Kontrolle) und einmal in 110 mL der neu entwickelten Additivlösung UG65 (Test) aufgeschwemmt. Die Abreicherung der Leukozyten erfolgte mittels Filtration dieser Erythrozytenkonzentrate durch einen herkömmlichen Leukozytendepletionsfilter. Nach der Filtration wurde das jeweilige Erythrozytenkonzentrat mit der gleichen Menge der jeweiligen Additivlösung (w/w) versetzt. 600 g des verdünnten Erythrozytenkonzentrates wurden in einen UVC-durchlässigen Bestrahlungsbeutel transferiert. Die UVC-Bestrahlung erfolgte wie oben beschrieben. Anschließend wurde aus dem verdünnten Erythrozytenkonzentrat mittels Zentrifugation und automatischer Separation ein herkömmliches Erythrozytenkonzentrat gewonnen. Die fertigen Erythrozytenkonzentrate (n=3, Test und Kontrolle) wurden bei 4 ± 2°C gelagert und wöchentlich wurden Proben zur Bestimmung der in vitro Qualität entnommen.

### Ergebnisse

Nach Herstellung hatten die Test- und Kontroll- Erythrozytenkonzentrate vergleichbare Werte für Volumen, Hkt und Hämoglobin pro Einheit (Tabelle 1).

**Tabelle 1: Herstellungsdaten UVC-bestrahlter Erythrozytenkonzentrate (EKs) in UG65 und SAG-M (n=3)**

| | Kontrolle (UVC-EK in SAG-M) | Test (UVC-EK in UG65) |
|---|---|---|
| Volumen [mL] | 277 ± 12 | 285 ± 15 |
| Hämatokrit [L/L] | 0,55 ± 0,02 | 0,56 ± 0,02 |
| Hämoglobin pro Einheit [g/Einheit] | 51,8 ± 4,0 | 50,9 ± 4,6 |

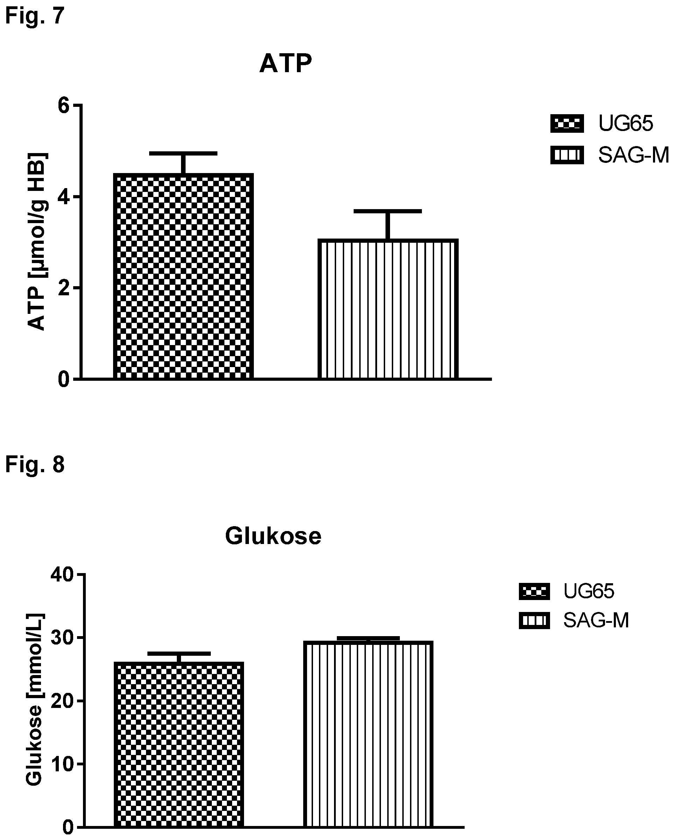

Als wichtigster Qualitätsparameter für Erythrozytenkonzentrate war die Hämolyserate der in UG65 UVC-bestrahlten und gelagerten Erythrozytenkonzentrate deutlich erniedrigt im Vergleich zu den in herkömmlicher Additivlösung SAG-M UVC-bestrahlten und gelagerten Erythrozytenkonzentraten (Fig. 6). Im Verlauf der Lagerung zeigten auch alle weiteren Qualitätsparameter signifikante Unterschiede zwischen den Kontroll- und Test-Erythrozytenkonzentraten (Fig. 7-9).

**Fig. 6** zeigt die Hämolyserate im Verlauf der Lagerung von EKs UVC-bestahlt und gelagert in der gleichen Additivlösung.

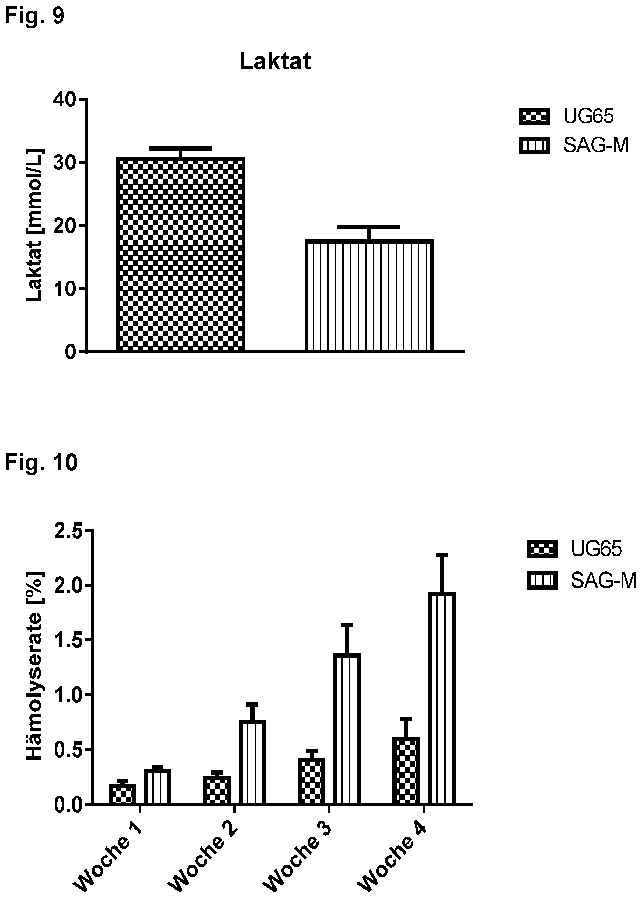

**Fig. 7** zeigt den ATP-Gehalt, **Fig.8** den Glukose-Gehalt und **Fig.9** den Laktat-Gehalt, jeweils am Ende der Lagerung (Woche 5) von EKs UVC-bestahlt und gelagert mit der gleichen Additivlösung.

Im Verlauf der Lagerung zeigte sich ein deutlicher Vorteil der neu entwickelten Additivlösung UG65 für die UVC-Bestrahlung von Erythrozytenkonzentraten. Die in UG65 UVC-bestrahlten und in UG65 gelagerten EKs waren hinsichtlich der Qualität denen in der konventionellen Additivlösung SAG-M überlegen.

### Versuch 4

### Qualität von Erythrozytenkonzentraten nach UVC-Bestrahlung auf der Stufe der Vollblute, Vergleich der Additivlösung UG65 mit konventioneller Additivlösung SAG-M

Vollblutspenden (ca. 570 mL) wurden wie oben beschrieben mit UVC bestrahlt und anschließend mittels automatischer Komponententrennung durch einen Abpressautomaten als "trockenes" Erythrozytenkonzentrat gewonnen (Hämatokrit > 0,8). Zwei trockene Erythrozytenkonzentrate wurden gepoolt und wieder aufgesplittet und anschließend einmal in 110 mL der handelsüblichen Additivlösung SAG-M (Kontrolle) und einmal in 110 mL der neu entwickelten Lösung UG65 (Test) aufgeschwemmt. Die Abreicherung der Leukozyten erfolgte mittels Filtration dieser Erythrozytenkonzentrate durch einen herkömmlichen Leukozytendepletionsfilter. Die Erythrozytenkonzentrate (n=4, Test und Kontrolle) wurden anschließend bei 4 ± 2°C gelagert und wöchentlich wurden Proben zur Bestimmung der in vitro Qualität entnommen.

### Ergebnisse

Nach Herstellung hatten die Test- und Kontroll- Erythrozytenkonzentrate vergleichbare Werte für Volumen, Hkt und Hämoglobin pro Einheit (Tabelle 2).

**Tabelle 2: Herstellungsdaten von aus UVC-bestrahltem Vollblut hergestellten Erythrozytenkonzentraten (EKs) gelagert in UG65 oder SAG-M (n=4)**

| | Kontrolle (UVC-EK in SAG-M) | Test (UVC-EK in UG65) |
|---|---|---|
| Volumen [mL] | 282 ± 12 | 284 ± 13 |
| Hämatokrit [L/L] | 0,56 ± 0,01 | 0,59 ± 0,02 |
| Hämoglobin pro Einheit [g/Einheit] | 53,8 ± 4,1 | 55,2 ± 4,0 |

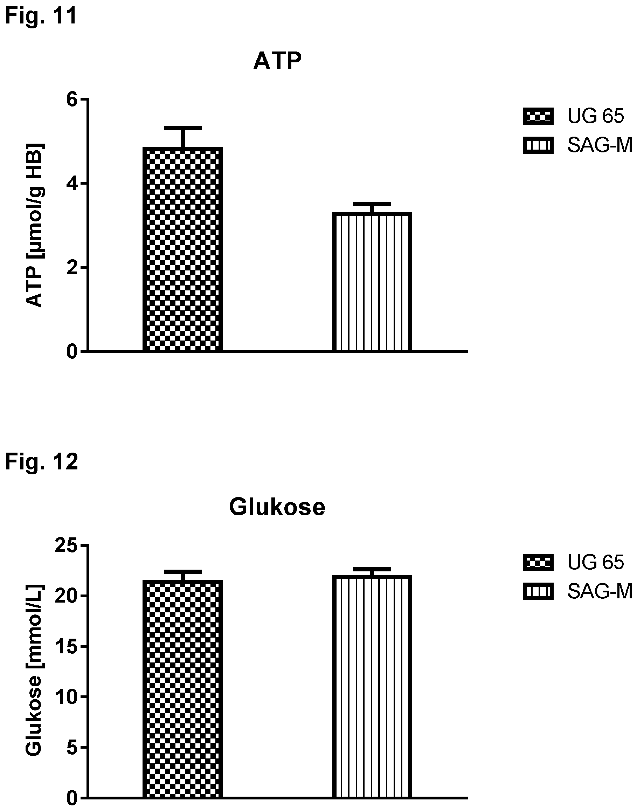

Als wichtigster Qualitätsparameter für Erythrozytenkonzentrate war die Hämolyserate der aus UVC-bestrahltem Vollblut gewonnenen Erythrozytenkonzentrate in UG65 deutlich erniedrigt im Vergleich zu den aus UVC-bestrahltem Vollblut gewonnenen Erythrozytenkonzentraten in der herkömmlichen Additivlösung SAG-M (Fig. 10). Im Verlauf der Lagerung zeigten auch weitere Qualitätsparameter signifikante Unterschiede zwischen den Kontroll- und Test- Erythrozytenkonzentraten (Fig 11 - 13).

**Fig.10** zeigt die Hämolyserate im Verlauf der Lagerung von EKs gewonnen aus UVC-bestrahltem Vollblut, gelagert in UG65 und SAG-M.

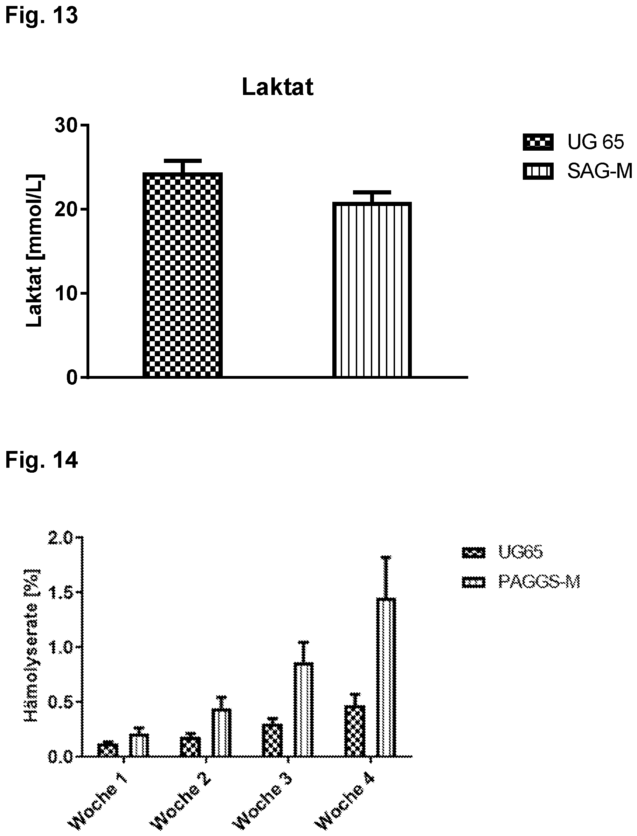

**Fig. 11** zeigt den ATP-Gehalt, **Fig.12** den Glukose-Gehalt und **Fig.13** den Laktat-Gehalt, jeweils am Ende der Lagerung (Woche 4).

Im Verlauf der Lagerung zeigte sich ein deutlicher Vorteil der neu entwickelten Additivlösung UG65 für die Gewinnung von Erythrozytenkonzentraten aus UVC-bestrahltem Vollblut. Die Qualität von EKs aus UVC-bestrahltem Vollblut in UG65 ist denen in der konventionellen Additivlösung SAG-M überlegen.

### Versuch 5

### Bakterieninaktivierung

Die Bakterienstämme Klebsiella pneumoniae (PEI-B-P-08-01), Serratia marcescens (PEI-B-P-56) und Pseudomonas fluorescens (PEI-B-P-77) wurden in CASA Bouillon vermehrt, mit humanem Serumalbumin versetzt und bis zur Verwendung eingefroren gelagert (siehe U. Gravemann, et al., Bacterial inactivation of platelet concentrates with the THERAFLEX UV-Platelets pathogen inactivation system. Transfusion, 2019. 59(4): p. 1324-1332.). Vollblut oder verdünnte Erythrozytenkonzentrate mit einem Hkt von ca. 0,3 wurden mit ca. 1 x 10⁶ KBE/mL Bakteriensuspension gespikt (n=3 für jedes verwendete Bakterium) und anschließend mit UVC-Licht und unter Schütteln bestrahlt. Anschließend wurde aus dem verdünnten Erythrozytenkonzentrat oder dem Vollblut ein herkömmliches Erythrozytenkonzentrat gewonnen. Zu unterschiedlichen Zeitpunkten wurden Proben entnommen und der Bakterientiter wurde durch Ausplatttieren auf Agarplatten bestimmt.

### Ergebnisse

Die Bakterien wurden sowohl in Erythrozytenkonzentraten (Tabelle 3) als auch im Vollblut (Tabelle 4) dosisabhängig inaktiviert mit log-Reduktionsfaktoren zwischen 4 und 6 log Stufen. Die Versuche belegen die Bakterien-Inaktivierungseffizienz des Verfahrens.

**Tabelle 3: Bakterieninaktivierung in Erythrozytenkonzentrat (Titer in KBE/mL, MW, n=3), mit erfindungsgemäßer Additivlösung UG65**

| | Klebsiella pneumoniae (PEI-B-P-08-01) | Serratia marcescens (PEI-B-P-56) | Pseudomonas fluorescens (PEI-B-P-77) |
|---|---|---|---|
| MW = Mittelwert | MW | MW | MW |
| 0,0 J/cm² | 1,6E+06 | 2,4E+06 | 9,9E+05 |
| 4,5 J/cm² | 12,8 | 2,7 | ≤ 1,5 |
| EK nach Wiederaufkonzentrieren | 10,3 | 3,7 | ≤ 1,5 |

**Tabelle 4: Bakterieninaktivierung in Vollblut (Titer in KBE/mL, MW, n=3), ohne Additivlösung während der UV-Bestrahlung**

| | Klebsiella pneumoniae (PEI-B-P-08-01) | Serratia marcescens (PEI-B-P-56) | Pseudomonas fluorescens (PEI-B-P-77) |
|---|---|---|---|
| MW = Mittelwert | MW | MW | MW |
| 0,0 J/cm² | 1,2E+06 | 1,2E+06 | 1,2E+06 |
| 6,0 J/cm² | 125,3 | 2,4 | 10,2 |
| EK | 1,8 | 1,3 | 15,8 |

### Versuch 6

### Virusinaktivierung

EMCV (strain EMC, ATCC VR129B), Sindbis Virus (Strain Ar339, ATCC VR-68) und VSV (Strain Indiana, ATCC VR-158) wurden auf Verozellen (African Green monkey cell line from kidney tissue, ATCC, Bio Whittaker No. BE76-108B) vermehrt und titriert. Die Anzucht und Titration erfolgte wie bei Mohr et al beschrieben (H. Mohr et al., A novel approach to pathogen reduction in platelet concentrates using short-wave ultraviolet light. Transfusion, 2009. 49(12): p. 2612-24).

Vollblut oder in der Additivlösung UG65 verdünnte Erythrozytenkonzentrate mit einem Hkt von ca. 0,3 wurden mit Virussuspension gespikt (10% v/v, n=3 für jedes verwendete Virus) und anschließend mit UVC-Licht und unter Schütteln bestrahlt. Anschließend wurde aus dem verdünnten Erythrozytenkonzentrat oder dem Vollblut ein herkömmliches Erythrozytenkonzentrat gewonnen. Zu unterschiedlichen Zeitpunkten wurden Proben entnommen und der Virustiter durch Endpunkttitration bestimmt. Bei Erreichen der Detektionsgrenze wurde statt der Endpunkttitration das Large Volume Plating angewandt.

Die Viren wurden sowohl in Erythrozytenkonzentrat (Tabelle 5) als auch im Vollblut (Tabelle 6) dosisabhängig inaktiviert mit log-Reduktionsfaktoren zwischen 3 und 5 log Stufen. Die Versuche belegen die Virus-Inaktivierungseffizienz des Verfahrens.

**Tabelle 5: Virusinaktivierung in Erythrozytenkonzentrat (log₁₀TCID₅₀, MW, n=3) mit erfindungsgemäßer Additivlösung UG65**

| | VSV | EMCV | Sindbis |
|---|---|---|---|
| | MW | MW | MW |
| 0 J/cm² | 8,43 | 6,82 | 6,92 |
| 4,5 J/cm² | ≤ 3,54 | 3,68 | 2,62 |
| EK nach Wiederaufkonzentrieren | ≤ 3,54 | 3,76 | 2,65 |

**Tabelle 6: Virusinaktivierung in Vollblut (log₁₀TCID₅₀, MW, n=3), ohne Additivlösung während der UV-Bestrahlung**

| | VSV | EMCV | Sindbis |
|---|---|---|---|
| | MW | MW | MW |
| 0,0 J/cm² | 8,19 | 6,86 | 6,64 |
| 6,0 J/cm² | 2,64 | 3,82 | 3,58 |
| EK | 2,49 | 3,80 | 2,49 |

### Versuch 7

### Qualität von Erythrozytenkonzentraten nach UVC-Bestrahlung auf der Stufe der Vollblute, Vergleich der Additivlösung UG65 mit der Additivlösung PAGGS-M

Zwei Vollblutspenden (jeweils ca. 500 mL Vollblut + 70 mL CPD Stabilisatorlösung) wurden gepoolt und wieder aufgeteilt. Die Vollblute wurden wie oben beschrieben mit UVC bestrahlt und anschließend mittels automatischer Komponententrennung durch einen Abpressautomaten als "trockenes" Erythrozytenkonzentrat gewonnen (Hämatokrit > 0,8).

Zwei trockene Erythrozytenkonzentrate wurden gepoolt und wieder aufgesplittet und anschließend einmal in 110 mL einer handelsüblichen Additivlösung PAGGS-M wie vorne beschrieben (Kontrolle, Zusammensetzung wie weiter vorne beschrieben) und einmal in 110 mL der neu entwickelten Lösung UG65 (Test) aufgeschwemmt. Die Abreicherung der Leukozyten erfolgte mittels Filtration dieser Erythrozytenkonzentrate durch einen herkömmlichen Leukozytendepletionsfilter. Die Erythrozytenkonzentrate (n=4, Test und Kontrolle) wurden anschließend bei 4 ± 2°C gelagert und wöchentlich wurden Proben zur Bestimmung der in vitro Qualität entnommen. Die Erythrozytenkonzentrate unterscheiden sich bei Verwendung der gleichen Menge CPD in Ihre Zusammensetzung zumindest jeweils dadurch, dass die Erythrozytenkonzentrate additiviert nach der Erfindung kein Mannitol aufwiesen und der Gehalt an Dinatriumhydrogenphosphat und Tri-Natriumcitrat deutlich größer war in den Erythrozytenkonzentraten nach der Erfindung verglichen mit solchen die neben CPD auch PAGGS-M enthielten.

**Tabelle 7: Konzentration im EK mit Additivlösung UG65**

| | | |
|---|---|---|
| D-Glukose | mmol/l | 18,6 |
| Natriumdihydrogenphosphat Dihydrat | mmol/l | 0,03 |
| Dinatriumhydrogenphosphat | mmol/l | 8,1 |
| Adenin | mmol/l | 0,7 |
| Guanosin | mmol/l | 0,5 |
| Natriumchlorid | mmol/l | 14,2 |
| Tri-Natriumcitrat | mmol/l | 10,3 |
| Citronensäure Monohydrat | mmol/l | 0,03 |

**Tabelle 8: Konzentration im EK mit Additivlösung PAGGS-M (Verwendung nicht erfindungsgemäss)**

| | | |
|---|---|---|
| D-Glukose | mmol/l | 17,1 |
| Natriumdihydrogenphosphat Dihydrat | mmol/l | 2,9 |
| Dinatriumhydrogenphosphat | mmol/l | 2,9 |
| Adenin | mmol/l | 0,5 |
| Guanosin | mmol/l | 0,5 |
| Natriumchlorid | mmol/l | 25,6 |
| Tri-Natriumcitrat | mmol/l | 0,2 |
| Citronensäure Monohydrat | mmol/l | 0,03 |
| Mannitol | mmol/l | 19,5 |

### Ergebnisse

Nach Herstellung hatten die Test- und Kontroll- Erythrozytenkonzentrate vergleichbare Werte für Volumen, Hkt und Hämoglobin pro Einheit (Tabelle 9).

**Tabelle 9: Herstellungsdaten von aus UVC-bestrahltem Vollblut hergestellten Erythrozytenkonzentraten (EKs) gelagert in UG65 oder PAGGS-M (n=4)**

| | Kontrolle (UVC-EK in PAGGS-M) | Test (UVC-EK in UG65) |
|---|---|---|
| Volumen [mL] | 266 ± 17 | 268 ± 15 |
| Hämatokrit [L/L] | 0,57 ± 0,01 | 0,58 ± 0,00 |
| Hämoglobin pro Einheit [g/Einheit] | 56,1 ± 5,0 | 56,9 ± 4,8 |

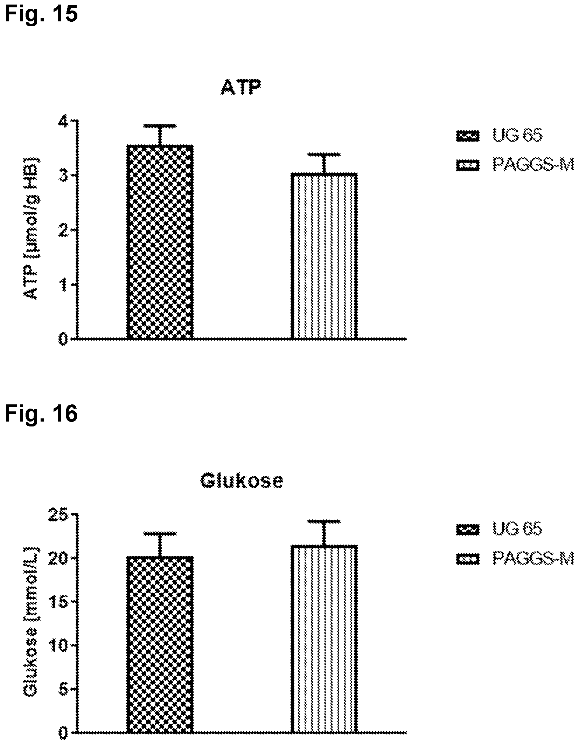

Als wichtigster Qualitätsparameter für Erythrozytenkonzentrate war die Hämolyserate der aus UVC-bestrahltem Vollblut gewonnenen Erythrozytenkonzentrate in UG65 deutlich erniedrigt im Vergleich zu den aus UVC-bestrahltem Vollblut gewonnenen Erythrozytenkonzentraten in der herkömmlichen Additivlösung PAGGS-M (Fig. 14). Im Verlauf der Lagerung zeigten auch weitere Qualitätsparameter signifikante Unterschiede zwischen den Kontroll- und Test- Erythrozytenkonzentraten (Fig. 15 bis 17).

**Fig. 14** zeigt die Hämolyserate im Verlauf der Lagerung von EKs gewonnen aus UVC-bestrahltem Vollblut, gelagert in UG65 und PAGGS-M.

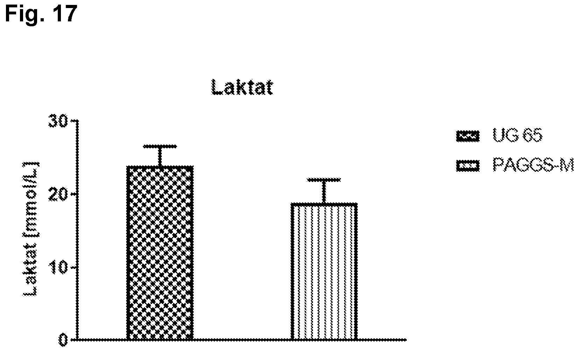

**Fig. 15** zeigt den ATP-Gehalt, **Fig. 16** den Glukose-Gehalt und **Fig. 17** den Laktat-Gehalt, jeweils am Ende der Lagerung (Woche 4).

Im Verlauf der Lagerung zeigte sich ein deutlicher Vorteil der neu entwickelten Additivlösung UG65 für die Gewinnung von Erythrozytenkonzentraten aus UVC-bestrahltem Vollblut. Die Qualität von EKs aus UVC-bestrahltem Vollblut in UG65 ist denen in der Additivlösung PAGGS-M überlegen.

## Patentansprüche

1. Verfahren zur Herstellung von Erythrozytenkonzentraten, wobei das Erythrozytenkonzentrat Erythrozyten enthält, die UV bestrahlt wurden, und das Verfahren die folgenden Schritte umfasst:
- Bestrahlung von Vollblut oder verdünntem Vollblut mit UV-Strahlung, Gewinnung eines Erythrozytenkonzentrates aus dem so bestrahlten Vollblut unter Zusatz einer Additivlösung oder Komponenten;
oder
- Bestrahlung eines Erythrozytenkonzentrates oder eines verdünnten Erythrozytenkonzentrates mit einem Hkt kleiner 0,5, das nach der UV-Bestrahlung auf einen Hkt größer oder gleich 0,5 aufkonzentriert wird, umfassend jeweils eine Additivlösung oder Komponenten;
oder
- Bestrahlung eines verdünnten Erythrozytenkonzentrates, umfassend eine zweite Additivlösung, wobei die zweite Additivlösung gegen eine Additivlösung oder Komponenten nach der Bestrahlung zumindest zu größer 75 Gew.%, bezogen auf die zweite Additivlösung, zumindest teilweise ausgetauscht wird;
wobei die UV-Bestrahlung jeweils mit einer Wellenlänge von 300 bis 200 nm erfolgt; wobei
nach einer Alternative A
die Additivlösung eine Additivlösung ist, umfassend neben Wasser zumindest die folgenden Komponenten:
12 bis 50 mmol/L Dinatriumhydrogenphosphat;
0,1 bis 3,5 mmol/L Adenin;
10 bis 90 mmol/L D-Glukose;
0,1 bis 3 mmol/L Guanosin;
10 bis 80 mmol/L Natriumchlorid; und
10 bis 50 mmol/L Tri-Natriumcitrat;
oder nach einer Alternative B
die Komponenten die folgenden Komponenten der Additivlösung sind:
Dinatriumhydrogenphosphat; Adenin; D-Glukose; Guanosin; Natriumchlorid; und
Tri-Natriumcitrat,
und die Komponenten nach Alternative B in einer solchen Menge eingesetzt werden, um im Ergebnis das Erythrozytenkonzentrat aufweisend
10 bis 30 mmol/I D-Glukose;
5 bis 12 mmol/I Dinatriumhydrogenphosphat;
0,3 bis 1,2 mmol/L Adenin;
0,25 bis 0,9 mmol/L Guanosin;
8 bis 25 mmol/L Natriumchlorid;
6 bis 18 mmol/L Tri-Natriumcitrat;
zu erhalten.

2. Verfahren nach Anspruch 1, wobei die Konzentration von Dinatriumhydrogenphosphat, Adenin, D-Glukose, Guanosin, Natriumchlorid und/oder Tri-Natriumcitrat jeweils einzeln oder gemeinsam in der Additivlösung folgende ist:
17 bis 50 mmol/L, insbesondere 20 bis 25 mmol/L, Dinatriumhydrogenphosphat;
1,5 bis 2,5 mmol/L Adenin;
45 bis 55 mmol/L D-Glukose;
1,25 bis 1,75 mmol/L Guanosin;
20 bis 60 mmol/L Natriumchlorid, insbesondere 35 bis 45 mmol/L Natriumchlorid;
14 bis 50 mmol/L, insbesondere 25 bis 35 mmol/L, Tri-Natriumcitrat.

3. Verfahren nach zumindest einem der Ansprüche 1 oder 2, wobei die Additivlösung aus den angegebenen Komponenten in den angegebenen Konzentrationen besteht, jeweils mit Rest Wasser.

4. Verfahren nach zumindest einem der vorhergehenden Ansprüche, wobei die Additivlösung durch eines oder mehrere der folgenden Merkmale gekennzeichnet ist:
a) die Additivlösung weist einen pH von größer 7, vorzugsweise größer 7,5, insbesondere einen pH von 8 bis 9, jeweils bei 22°C auf;
b) die Osmolalität der Additivlösung beträgt 260 bis 300 mOsm/kg;
c) die Additivlösung enthält
- kein Mannitol oder
- kein Sorbitol oder
- kein Mannitol und kein Sorbitol.

5. Verfahren nach zumindest einem der vorhergehenden Ansprüche, wobei das Erythrozytenkonzentrat aufweist:
0,40 bis 0,80 L/L, vorzugsweise 0,50 bis 0,70 L/L, Erythrozyten und
0,10 bis 0,60 L/L Additivlösung, vorzugsweise 0,25 bis 0,50 L/L, Additivlösung,
wobei sich die Summe der Volumenanteile in L/L jeweils zu einem Zahlenwert von 1 oder kleiner 1 L/L ergänzt.

6. Verfahren nach zumindest einem der vorhergehenden Ansprüche, wobei das Erythrozytenkonzentrat weiterhin aufweist:
0,0001 - 0,1 L/L Stabilisatorlösung, insbesondere CPD-Stabilisatorlösung, und/oder
0,0001 bis 0,2 L/L Humanplasma.

7. Verfahren nach zumindest einem der vorhergehenden Ansprüche, wobei das Erythrozytenkonzentrat Erythrozyten enthält, die auf der Stufe des Vollblutes UV bestrahlt wurden.

8. Verfahren nach zumindest einem der Ansprüche 1 bis 6, wobei das Erythrozytenkonzentrat Erythrozyten enthält, die auf der Stufe eines verdünnten Erythrozytenkonzentrates mit UV-Strahlung bestrahlt wurden und das verdünnte Erythrozytenkonzentrat vorzugswiese einen Hkt kleiner 0,5 aufweist und nach der UV-Bestrahlung aufkonzentriert wird.

9. Verfahren nach zumindest einem der vorhergehenden Ansprüche, wobei das Erythrozytenkonzentrat durch eines oder mehrere der folgenden Merkmale gekennzeichnet ist:
a) das Erythrozytenkonzentrat weist einen Hkt von 0,4 bis 0,8, insbesondere 0,5 bis 0,7, auf;
b) das Erythrozytenkonzentrat enthält
- kein Mannitol oder
- kein Sorbitol oder
- kein Mannitol und kein Sorbitol.
c) das Erythrozytenkonzentrat weist nach Alternative A auf:
10 bis 30 mmol/l, insbesondere 14 bis 26 mmol/l, D-Glukose;
5 bis 12 mmol/l, insbesondere 6 bis 10 mmol/l, Dinatriumhydrogenphosphat;
0,3 bis 1,2 mmol/L, insbesondere 0,5 bis 0,8 mmol/l, Adenin;
0,25 bis 0,9 mmol/L, insbesondere 0,4 bis 0,7 mmol/L, Guanosin;
8 bis 25 mmol/L, insbesondere 11 bis 20 mmol/L, Natriumchlorid;
6 bis 18 mmol/L, insbesondere 8 bis 16 mmol/L, Tri-Natriumcitrat;
und fakultativ
0,01 bis 1 mmol/L, insbesondere 0,02 bis 0,8 mmol/L, Natriumdihydrogenphosphat; und
0,01 bis 1 mmol/L, insbesondere 0,02 bis 0,8 mmol/L, Citronensäure.
oder nach Alternative B:
14 bis 26 mmol/I D-Glukose;
6 bis 10 mmol/I Dinatriumhydrogenphosphat;
0,5 bis 0,8 mmol/l Adenin;
0,4 bis 0,7 mmol/L Guanosin;
11 bis 20 mmol/L Natriumchlorid; und
8 bis 16 mmol/L Tri-Natriumcitrat;
und fakultativ
0,01 bis 1 mmol/L, insbesondere 0,02 bis 0,8 mmol/L, Natriumdihydrogenphosphat; und
0,01 bis 1 mmol/L, insbesondere 0,02 bis 0,8 mmol/L, Citronensäure;
d) das Erythrozytenkonzentrat weist weniger als 0,6 mmol/L, insbesondere weniger als 0,5 mmol/L Natriumdihydrogenphosphat auf.

10. Verfahren nach zumindest einem der vorhergehenden Ansprüche, wobei das Verfahren die folgenden Schritte umfasst:
- Bestrahlung eines Erythrozytenkonzentrates, umfassend die Additivlösung oder die Komponenten wobei das Erythrozytenkonzentrat ein verdünntes Erythrozytenkonzentrat mit einem Hkt kleiner 0,5 ist und nach der UV-Bestrahlung auf einen Hkt größer oder gleich 0,5 aufkonzentriert wird;
oder
- Bestrahlung eines verdünnten Erythrozytenkonzentrates, umfassend eine zweite Additivlösung, wobei das verdünnte Erythrozytenkonzentrat einen Hkt kleiner 0,5 aufweist und nach der UV-Bestrahlung auf einen Hkt größer 0,5 aufkonzentriert wird, wobei die zweite Additivlösung gegen die Additivlösung oder die Komponenten nach der Bestrahlung zumindest zu größer 75 Gew.%, bezogen auf die zweite Additivlösung, zumindest teilweise ausgetauscht wird, vorzugsweise im Wesentlichen vollständig ausgetauscht wird.

11. Verfahren nach zumindest einem der vorhergehenden Ansprüche, wobei die UV-Bestrahlung jeweils mit einer Wellenlänge von 280 bis 220 nm und bevorzugt 260 bis 240 nm erfolgt.

12. Verfahren nach zumindest einem der vorhergehenden Ansprüche, wobei die Additivlösung oder die Komponenten zu einem Erythrozytenkonzentrat mit einem Hkt von größer gleich 0,5 zugegeben wird.

13. Verwendung einer Additivlösung zur Lagerung von Erythrozytenkonzentraten, wobei die Erythrozytenkonzentrate Erythrozyten enthalten, die UV bestrahlt wurden,
wobei die Additivlösung neben Wasser zumindest die folgenden Komponenten umfasst:
12 bis 50 mmol/L Dinatriumhydrogenphosphat;
0,1 bis 3,5 mmol/L Adenin;
10 bis 90 mmol/L D-Glukose;
0,1 bis 3 mmol/L Guanosin;
10 bis 80 mmol/L Natriumchlorid; und
10 bis 50 mmol/L Tri-Natriumcitrat.

14. Verwendung nach Anspruch 13, wobei die Konzentration von Dinatriumhydrogenphosphat, Adenin, D-Glukose, Guanosin, Natriumchlorid und/oder Tri-Natriumcitrat jeweils einzeln oder gemeinsam folgende ist:
17 bis 50 mmol/L, insbesondere 20 bis 25 mmol/L, Dinatriumhydrogenphosphat;
1,5 bis 2,5 mmol/L Adenin;
45 bis 55 mmol/L D-Glukose;
1,25 bis 1,75 mmol/L Guanosin;
20 bis 60 mmol/L Natriumchlorid, insbesondere 35 bis 45 mmol/L Natriumchlorid;
14 bis 50 mmol/L, insbesondere 25 bis 35 mmol/L, Tri-Natriumcitrat.

15. Verwendung nach Anspruch 13 oder 14, wobei die Additivlösung durch eines oder mehrere der folgenden Merkmale gekennzeichnet ist:
a) die Additivlösung besteht aus den angegebenen Komponenten in den angegebenen Konzentrationen, jeweils mit Rest Wasser;
b) die Additivlösung weist einen pH von größer 7, vorzugsweise größer 7,5, insbesondere einen pH von 8 bis 9, jeweils bei 22°C auf;
c) die Osmolalität der Additivlösung beträgt 260 bis 300 mOsm/kg;
d) die Additivlösung enthält
- kein Mannitol,
- kein Sorbitol oder
- kein Mannitol und kein Sorbitol.

## Claims

1. A method for the production of erythrocyte concentrates, wherein the erythrocyte concentrate comprises erythrocytes, which were UV-irradiated, and the method comprises the following steps:
- irradiation of whole blood or diluted whole blood with UV radiation, obtaining an erythrocyte concentrate from the whole blood so irradiated by adding an additive solution or components;
or
- irradiation of an erythrocyte concentrate or of a diluted erythrocyte concentrate with an hct of less than 0.5, which is concentrated to an hct of greater than or equal to 0.5 after the UV irradiation, each comprising an additive solution or components;
or
- irradiation of a diluted erythrocyte concentrate, comprising a second additive solution, wherein the second additive solution is at least partially replaced with an additive solution or components after the irradiation at least at greater than 75% by weight, based on the second additive solution;
wherein the UV irradiation in each case is conducted at a wavelength of 300 to 200 nm; wherein
according to an alternative A
the additive solution is an additive solution comprising along with water at least the following components:
12 to 50 mmol/L of disodium hydrogen phosphate;
0.1 to 3.5 mmol/L of adenine;
10 to 90 mmol/L of D-glucose;
0.1 to 3 mmol/L of guanosine;
10 to 80 mmol/L of sodium chloride; and
10 to 50 mmol/L of trisodium citrate,
or according to an alternative B
the components are the following components of the additive solution:
disodium hydrogen phosphate; adenine; D-glucose; guanosine; sodium chloride; and trisodium citrate,
and the components according to alternative B are used in such a quantity in order to obtain, as a result, the erythrocyte concentrate comprising
10 to 30 mmol/L of D-glucose;
5 to 12 mmol/L of disodium hydrogen phosphate;
0.3 to 1.2 mmol/L of adenine;
0.25 to 0.9 mmol/L of guanosine;
8 to 25 mmol/L of sodium chloride;
6 to 18 mmol/L of trisodium citrate.

2. The method according to claim 1, wherein the concentration in the additive solution of disodium hydrogen phosphate, adenine, D-glucose, guanosine, sodium chloride and/or trisodium citrate is, each individually or jointly, the following:
17 to 50 mmol/L, in particular 20 to 25 mmol/L, of disodium hydrogen phosphate;
1.5 to 2.5 mmol/L of adenine;
45 to 55 mmol/L of D-glucose;
1.25 to 1.75 mmol/L of guanosine;
20 to 60 mmol/L of sodium chloride, in particular 35 to 45 mmol/L of sodium chloride;
14 to 50 mmol/L, in particular 25 to 35 mmol/L, of trisodium citrate.

3. The method according to at least any one of claims 1 or 2, wherein the additive solution consists of the specified components in the specified concentrations, each with the remainder being water.

4. The method according to at least any one of the preceding claims, wherein the additive solution is **characterized by** one or more of the following:
a) the additive solution has a pH of greater than 7, preferably greater than 7.5, in particular a pH of 8 to 9, each at 22°C;
b) the osmolality of the additive solution is 260 to 300 mOsm/kg;
c) the additive solution comprises
- no mannitol or
- no sorbitol or
- no mannitol and no sorbitol.

5. The method according to at least any one of the preceding claims, wherein the erythrocyte concentrate comprises:
0.40 to 0.80 L/L, preferably 0.50 to 0.70 L/L, of erythrocytes and
0.10 to 0.60 L/L of additive solution, preferably 0.25 to 0.50 L/L, of additive solution, wherein the sum of the proportions by volume in L/L in each case adds up to a numerical value of 1 or less than 1 L/L.

6. The method according to at least any one of the preceding claims, wherein the erythrocyte concentrate furthermore comprises:
0.0001 - 0.1 L/L of stabilizer solution, in particular CPD stabilizer solution, and/or
0.0001 to 0.2 L/L of human plasma.

7. The method according to at least any one of the preceding claims, wherein the erythrocyte concentrate comprises erythrocytes, which were UV-irradiated at the whole blood stage.

8. The method according to at least any one of claims 1 to 6, wherein the erythrocyte concentrate comprises erythrocytes, which were irradiated with UV radiation at the stage of a diluted erythrocyte concentrate, and the diluted erythrocyte concentrate preferably has an hct of less than 0.5 and is concentrated after the UV irradiation.

9. The method according to at least any one of the preceding claims, wherein the erythrocyte concentrate is **characterized by** one or more of the following:
a) the erythrocyte concentrate has an hct of 0.4 to 0.8, in particular 0.5 to 0.7;
b) the erythrocyte concentrate comprises
- no mannitol or
- no sorbitol or
- no mannitol and no sorbitol;
c) the erythrocyte concentrate comprises according to alternative A:
10 to 30 mmol/L, in particular 14 to 26 mmol/L, of D-glucose;
5 to 12 mmol/L, in particular 6 to 10 mmol/L, of disodium hydrogen phosphate;
0.3 to 1.2 mmol/L, in particular 0.5 to 0.8 mmol/L, of adenine;
0.25 to 0.9 mmol/L, in particular 0.4 to 0.7 mmol/L, of guanosine;
8 to 25 mmol/L, in particular 11 to 20 mmol/L, of sodium chloride;
6 to 18 mmol/L, in particular 8 to 16 mmol/L, of trisodium citrate;
and optionally
0.01 to 1 mmol/L, in particular 0.02 to 0,8 mmol/L, of sodium dihydrogen phosphate; and
0.01 to 1 mmol/L, in particular 0.02 to 0.8 mmol/L, of citric acid;
or according to alternative B:
14 to 26 mmol/L of D-glucose;
6 to 10 mmol/L of disodium hydrogen phosphate;
0.5 to 0.8 mmol/L of adenine;
0.4 to 0.7 mmol/L of guanosine;
11 to 20 mmol/L of sodium chloride;
8 to 16 mmol/L of trisodium citrate;
and optionally
0.01 to 1 mmol/L, in particular 0.02 to 0.8 mmol/L, of sodium dihydrogen phosphate; and
0.01 to 1 mmol/L, in particular 0.02 to 0.8 mmol/L, of citric acid;
d) the erythrocyte concentrate has less than 0.6 mmol/L, in particular less than 0.5 mmol/L, of sodium dihydrogen phosphate.

10. The method according to at least any one of the preceding claims, wherein the method comprises the following steps:
- irradiation of an erythrocyte concentrate, comprising the additive solution or the components, wherein the erythrocyte concentrate is a diluted erythrocyte concentrate with an hct of less than 0.5 and is concentrated to an hct of greater than or equal to 0.5 after the UV irradiation;
or
- irradiation of a diluted erythrocyte concentrate, comprising a second additive solution, wherein the diluted erythrocyte concentrate has an hct of less than 0.5 and is concentrated to an hct of greater than 0.5 after the UV irradiation, wherein the second additive solution is at least partially replaced with the additive solution or the components after the irradiation at least at greater than 75% weight, based on the second additive solution, preferably replaced essentially completely.

11. The method according to at least any one of the preceding claims, wherein the UV irradiation in each case is conducted at a wavelength of 280 to 220 nm and preferably 260 to 240 nm.

12. The method according to at least any one of the preceding claims, wherein the additive solution or the components is/are added to an erythrocyte concentrate with an hct of greater than or equal to 0.5.

13. Use of an additive solution for storing erythrocyte concentrates, wherein the erythrocyte concentrates comprises erythrocytes, which were UV-irradiated, wherein the additive solution comprises along with water at least the following components:
12 to 50 mmol/L of disodium hydrogen phosphate;
0.1 to 3.5 mmol/L of adenine;
10 to 90 mmol/L of D-glucose;
0.1 to 3 mmol/L of guanosine;
10 to 80 mmol/L of sodium chloride; and
10 to 50 mmol/L of trisodium citrate.

14. The use according to claim 13, wherein the concentration of disodium hydrogen phosphate, adenine, D-glucose, guanosine, sodium chloride and/or trisodium citrate is, each individually or jointly, the following:
17 to 50 mmol/L, in particular 20 to 25 mmol/L, of disodium hydrogen phosphate;
1.5 to 2.5 mmol/L of adenine;
45 to 55 mmol/L of D-glucose;
1.25 to 1.75 mmol/L of guanosine;
20 to 60 mmol/L of sodium chloride, in particular 35 to 45 mmol/L of sodium chloride;
14 to 50 mmol/L, in particular 25 to 35 mmol/L, of trisodium citrate.

15. The use according to claim 13 or 14, wherein the additive solution is **characterized by** one or more of the following:
a) the additive solution consists of the specified components in the specified concentrations, each with the remainder being water;
b) the additive solution has a pH of greater than 7, preferably greater than 7.5, in particular a pH of 8 to 9, each at 22°C;
c) the osmolality of the additive solution is 260 to 300 mOsm/kg;
d) the additive solution comprises
- no mannitol,
- no sorbitol or
- no mannitol and no sorbitol.

## Revendications

1. Procédé de production de concentrés d'érythrocytes, le concentré d'érythrocytes contenant des érythrocytes, qui ont été irradiés par des UV, et le procédé comprenant les étapes suivantes de :
- irradiation de sang entier ou de sang entier dilué avec un rayonnement UV, obtention d'un concentré d'érythrocytes à partir du sang entier ainsi irradié en ajoutant une solution additive ou des composants ;
ou
- irradiation d'un concentré d'érythrocytes ou d'un concentré d'érythrocytes dilué ayant un HCT inférieur à 0,5, qui a été reconcentré après irradiation par les UV jusqu'à un HCT supérieur ou égal à 0,5, comprenant respectivement une solution additive ou des composants ;
ou
- irradiation d'un concentré d'érythrocytes dilué, comprenant une deuxième solution additive, la deuxième solution additive étant remplacée après l'irradiation au moins partiellement par une solution additive ou des composants à au moins plus de 75 % en poids, par rapport à la deuxième solution additive ;
l'irradiation par les UV s'effectuant respectivement à une longueur d'ondes de 300 à 200 nm ; où
selon une variante A
la solution additive est une solution additive comprenant, hormis de l'eau, au moins les composants suivants :
12 à 50 mmol/litre d'hydrogénophosphate disodique ;
0,1 à 3,5 mmol/litre d'adénine ;
10 à 90 mmol/litre de D-glucose ;
0,1 à 3 mmol/litre de guanosine ;
10 à 80 mmol/litre de chlorure de sodium ; et
10 à 50 mmol/litre de citrate trisodique ;
ou selon une variante B
les composants sont les composants suivants de la solution additive :
l'hydrogénophosphate disodique ; l'adénine ; le D-glucose ; la guanosine ; le chlorure de sodium ; et
le citrate trisodique,
et les composants selon la variante B sont mis en œuvre dans une quantité telle permettant d'obtenir au final le concentré d'érythrocytes présentant
10 à 30 mmol/litre de D-glucose ;
5 à 12 mmol/litre d'hydrogénophosphate disodique ;
0,3 à 1,2 mmol/litre d'adénine ;
0,25 à 0,9 mmol/litre de guanosine ;
8 à 25 mmol/litre de chlorure de sodium ;
6 à 18 mmol/litre de citrate trisodique.

2. Procédé selon la revendication 1, dans lequel la concentration en hydrogénophosphate disodique, adénine, D-glucose, guanosine, chlorure de sodium et/ou citrate trisodique est respectivement la suivante individuellement ou collectivement dans la solution additive :
17 à 50 mmol/litre, en particulier 20 à 25 mmol/litre d'hydrogénophosphate disodique ;
1,5 à 2,5 mmol/litre d'adénine ;
45 à 55 mmol/litre de D-glucose ;
1,25 à 1,75 mmol/litre de guanosine ;
20 à 60 mmol/litre de chlorure de sodium, en particulier 35 à 45 mmol/litre de chlorure de sodium ;
14 à 50 mmol/litre, en particulier 25 à 35 mmol/litre de citrate trisodique.

3. Procédé selon au moins l'une des revendications 1 ou 2, dans lequel la solution additive se compose des composants indiqués en les concentrations indiquées, respectivement avec un reste d'eau.

4. Procédé selon au moins l'une des revendications précédentes, dans lequel la solution additive est **caractérisée par** une ou plusieurs des caractéristiques suivantes :
a) la solution additive présente un pH supérieur à 7, de préférence supérieur à 7,5, en particulier un pH de 8 à 9, respectivement à 22°C ;
b) l'osmolalité de la solution additive est de 260 à 300 mOsm/kg ;
c) la solution additive ne contient
- pas de mannitol ou
- pas de sorbitol ou
- pas de mannitol ni de sorbitol.

5. Procédé selon au moins l'une des revendications précédentes, dans lequel le concentré d'érythrocytes présente :
0,40 à 0,80 litre/litre, de préférence 0,50 à 0,70 litre/litre d'érythrocytes et
0,10 à 0,60 litre/litre de solution additive, de préférence 0,25 à 0,50 litre/litre de solution additive,
où la somme des proportions volumiques en litre/litre s'additionne respectivement jusqu'à une valeur en nombre de 1 ou inférieure à 1 litre/litre.

6. Procédé selon au moins l'une des revendications précédentes, dans lequel le concentré d'érythrocytes présente en outre :
0,0001 à 0,1 litre/litre de solution stabilisante, en particulier de solution stabilisante CPD, et/ou
0,0001 à 0,2 litre/litre de plasma humain.

7. Procédé selon au moins l'une des revendications précédentes, dans lequel le concentré d'érythrocytes contient des érythrocytes qui ont été irradiés par des UV au stade de sang entier.

8. Procédé selon au moins l'une des revendications 1 à 6, dans lequel le concentré d'érythrocytes contient des érythrocytes qui ont été irradiés par un rayonnement UV au stade d'un concentré d'érythrocytes dilué et le concentré d'érythrocytes dilué présente un HCT de préférence inférieur à 0,5 et est reconcentré après l'irradiation par les UV.

9. Procédé selon au moins l'une des revendications précédentes, dans lequel le concentré d'érythrocytes est **caractérisé par** une ou plusieurs des caractéristiques suivantes :
a) le concentré d'érythrocytes présente un HCT de 0,4 à 0,8, en particulier de 0,5 à 0,7 ;
b) le concentré d'érythrocytes ne contient
- pas de mannitol ou
- pas de sorbitol ou
- pas de mannitol ni de sorbitol,
c) le concentré d'érythrocytes présente selon la variante A :
10 à 30 mmol/litre, en particulier 14 à 26 mmol/litre de D-glucose ;
5 à 12 mmol/litre, en particulier 6 à 10 mmol/litre d'hydrogénophosphate disodique ;
0,3 à 1,2 mmol/litre, en particulier 0,5 à 0,8 mmol/litre d'adénine ;
0,25 à 0,9 mmol/litre, en particulier 0,4 à 0,7 mmol/litre de guanosine ;
8 à 25 mmol/litre, en particulier 11 à 20 mmol/litre de chlorure de sodium ;
6 à 18 mmol/litre, en particulier 8 à 16 mmol/litre de citrate trisodique ;
et facultativement
0,01 à 1 mmol/litre, en particulier 0,02 à 0,8 mmol/litre de dihydrogénophosphate sodique ; et
0,01 à 1 mmol/litre, en particulier 0,02 à 0,8 mmol/litre d'acide citrique.
ou selon la variante B :
14 à 26 mmol/litre de D-glucose ;
6 à 10 mmol/litre d'hydrogénophosphate disodique ;
0,5 à 0,8 mmol/litre d'adénine ;
0,4 à 0,7 mmol/litre de guanosine ;
11 à 20 mmol/litre de chlorure de sodium ; et
8 à 16 mmol/litre de citrate trisodique ;
et facultativement
0,01 à 1 mmol/litre, en particulier 0,02 à 0,8 mmol/litre de dihydrogénophosphate sodique ; et
0,01 à 1 mmol/litre, en particulier 0,02 à 0,8 mmol/litre d'acide citrique ;
d) le concentré d'érythrocytes présente moins de 0,6 mmol/litre, en particulier moins de 0,5 mmol/litre de dihydrogénophosphate de sodium.

10. Procédé selon au moins l'une des revendications précédentes, ledit procédé comprenant les étapes suivantes de :
- irradiation d'un concentré d'érythrocytes, comprenant la solution additive ou les composants, le concentré d'érythrocytes étant un concentré d'érythrocytes dilué ayant un HCT inférieur à 0,5 qui a été reconcentré après irradiation par les UV jusqu'à un HCT supérieur ou égal à 0,5 ;
ou
- irradiation d'un concentré d'érythrocytes dilué, comprenant une deuxième solution additive, le concentré d'érythrocytes présentant un HCT inférieur à 0,5 et étant reconcentré après irradiation par les UV jusqu'à un HCT supérieur à 0,5, la deuxième solution additive étant remplacée après l'irradiation au moins partiellement par la solution additive ou les composants à au moins plus de 75 % en poids, par rapport à la deuxième solution additive, de préférence sensiblement entièrement remplacée.

11. Procédé selon au moins l'une des revendications précédentes, dans lequel l'irradiation par les UV s'effectue respectivement à une longueur d'ondes de 280 à 220 nm et de préférence 260 à 240 nm.

12. Procédé selon au moins l'une des revendications précédentes, dans lequel la solution additive ou les composants sont ajoutés à un concentré d'érythrocytes ayant un HCT supérieur ou égal à 0,5.

13. Utilisation d'une solution additive pour le stockage des concentrés d'érythrocytes, dans laquelle les concentrés d'érythrocytes contiennent des érythrocytes qui ont été irradiés par des UV,
dans laquelle la solution additive comprend, hormis de l'eau, au moins les composants suivants :
12 à 50 mmol/litre d'hydrogénophosphate disodique ;
0,1 à 3,5 mmol/litre d'adénine ;
10 à 90 mmol/litre de D-glucose ;
0,1 à 3 mmol/litre de guanosine ;
10 à 80 mmol/litre de chlorure de sodium ; et
10 à 50 mmol/litre de citrate trisodique ;

14. Utilisation selon la revendication 13, dans laquelle la concentration en hydrogénophosphate disodique, adénine, D-glucose, guanosine, chlorure de sodium et/ou citrate trisodique est respectivement la suivante individuellement ou collectivement :
17 à 50 mmol/litre, en particulier 20 à 25 mmol/litre d'hydrogénophosphate disodique ;
1,5 à 2,5 mmol/litre d'adénine ;
45 à 55 mmol/litre de D-glucose ;
1,25 à 1,75 mmol/litre de guanosine ;
20 à 60 mmol/litre de chlorure de sodium, en particulier 35 à 45 mmol/litre de chlorure de sodium ;
14 à 50 mmol/litre, en particulier 25 à 35 mmol/litre de citrate trisodique.

15. Utilisation selon la revendication 13 ou 14, dans laquelle la solution additive est **caractérisée par** une ou plusieurs des caractéristiques suivantes :
a) la solution additive se compose des composants indiqués en les concentrations indiquées, respectivement avec un reste d'eau ;
b) la solution additive présente un pH supérieur à 7, de préférence supérieur à 7,5, en particulier un pH de 8 à 9, respectivement à 22°C ;
c) l'osmolalité de la solution additive est de 260 à 300 mOsm/kg ;
d) la solution additive ne contient
- pas de mannitol,
- pas de sorbitol ou
- pas de mannitol ni de sorbitol.
